# EUROPEAN PATENT APPLICATION

(11) **EP 2 353 590 A1**
(43) Date of publication of application: **10.08.2011**
(21) Application number: 11001512.0
(22) Date of filing: 15.02.2006
(51) Int. Cl.: A61K 9/72, A61K 9/10, A61K 9/14

(54) **Aerosol and injectable formulations of nanoparticulate benzodiazepine**

(30) Priority: 15.02.2005 US 653034 P
(62) Divisional of application: 06735067.8
(71) Applicant: Elan Pharma International Limited, Athlone Westmeath (IE)
(72) Inventor: LIVERSIDGE, Gary, West Chester, Pennsylvania 19380 (US); JENKINS, Scott, Downington, Pennsylvania 19335 (US)
(74) Representative: Wichmann, Hendrik

(57) **Abstract**

Described are nanoparticulate formulations of a benzodiazepine, such as lorazepam, that does not require the presence of polyethylene glycol and propylene glycol as stabilizers, and methods of making and using such formulations. The formulations are particularly useful in aerosol and injectable dosage forms, and comprise nanoparticulate benzodiazepine, such as lorazepam, and at least one surface stabilizer. The formulations are useful in the treatment of status epilepticus, treatment of irritable bowel syndrome, sleep induction, acute psychosis, and as a pre-anesthesia medication.

## Description

This is a divisional application on the basis of the parent application European Patent Application 06 735 067.8 the content of which is incorporated herein by reference.

### FIELD OF THE INVENTION

The present invention is directed to aerosol and injectable formulations of nanoparticulate benzodiazepine, and preferably, nanoparticulate lorazepam. The compositions of the invention are useful in treating status epilepticus, sleep induction, acute psychosis, irritable bowel syndrome, and for pre-anesthesia medication. Also encompassed by the invention are methods of making and using such compositions.

### BACKGROUND OF THE INVENTION

### I. Administration Routes for Drugs

The route of administration of a drug substance can be critical to its pharmacological effectiveness. Various routes of administration exist, and all have their own advantages and disadvantages. Oral drug delivery of tablets, capsules, liquids, and the like is the most convenient approach to drug delivery, but many drug compounds are not amenable to oral administration. For example, modem protein drugs which are unstable in the acidic gastric environment or which are rapidly degraded by proteolytic enzymes in the digestive tract are poor candidates for oral administration. Similarly, poorly water soluble compounds which do not dissolve rapidly enough to be orally absorbed are likely to be ineffective when given as oral dosage forms. Oral administration can also be undesirable because drugs which are administered orally are generally distributed to all tissues in the body, and not just to the intended site of pharmacological activity. Alternative types of systemic administration are subcutaneous or intravenous injection. This approach avoids the gastrointestinal tract and therefore can be an effective route for delivery of proteins and peptides. However, these routes of administration have a low rate of patient compliance, especially for drugs such as insulin which must be administered one or more times daily. Additional alternative methods of drug delivery have been developed including transdermal, rectal, vaginal, intranasal, and pulmonary delivery.

Nasal drug delivery relies on inhalation of an aerosol through the nose so that active drug substance can reach the nasal mucosa. Drugs intended for systemic activity can be absorbed into the bloodstream because the nasal mucosa is highly vascularized. Alternatively, if the drug is intended to act topically, it is delivered directly to the site of activity and does not have to distribute throughout the body; hence, relatively low doses may be used. Examples of such drugs are decongestants, antihistamines, and anti-inflammatory steroids for seasonal allergic rhinitis.

Pulmonary drug delivery relies on inhalation of an aerosol through the mouth and throat so that the drug substance can reach the lung. For systemically active drugs, it is desirable for the drug particles to reach the alveolar region of the lung, whereas drugs which act on the smooth muscle of the conducting airways should preferentially deposit in the bronchiole region. Such drugs can include beta-agonists, anti cholinergics, and corticosteroids.

### A. Droplet/Particle Size Determines Deposition Site

In developing a therapeutic aerosol, the aerodynamic size distribution of the inhaled particles is the single most important variable in defining the site of droplet or particle deposition in the patient; in short, it will determine whether drug targeting succeeds or fails. *See* P. Byron, "Aerosol Formulation, Generation, and Delivery Using Nonmetered Systems," Respiratory Drug Delivery, 144-151, 144 (CRC Press, 1989). Thus, a prerequisite in developing a therapeutic aerosol is a preferential particle size. The deposition of inhaled aerosols involves different mechanisms for different size particles. D. Swift (1980); Parodi et al., "Airborne Particles and Their Pulmonary Deposition," in Scientific Foundations of Respiratory Medicine, Scaddings et al. (eds.), pp. 545-557 (W. B. Saunders, Philadelphia, 1981); J. Heyder, "Mechanism of Aerosol Particle Deposition," Chest, 80:820-823 (1981).

Generally, inhaled particles are subject to deposition by one of two mechanisms: impaction, which usually predominates for larger particles, and sedimentation, which is prevalent for smaller particles. Impaction occurs when the momentum of an inhaled particle is large enough that the particle does not follow the air stream and encounters a physiological surface. In contrast, sedimentation occurs primarily in the deep lung when very small particles which have traveled with the inhaled air stream encounter physiological surfaces as a result of random diffusion within the air stream. For intranasally administered drug compounds which are inhaled through the nose, it is desirable for the drug to impact directly on the nasal mucosa; thus, large (ca. 5 to 100 µm) particles or droplets are generally preferred for targeting of nasal delivery.

Pulmonary drug delivery is accomplished by inhalation of an aerosol through the mouth and throat. Particles having aerodynamic diameters of greater than about 5 microns generally do not reach the lung; instead, they tend to impact the back of the throat and are swallowed and possibly orally absorbed. Particles having diameters of about 2 to about 5 microns are small enough to reach the upper- to mid-pulmonary region (conducting airways), but are too large to reach the alveoli. Even smaller particles, *i.e.,* about 0.5 to about 2 microns, are capable of reaching the alveolar region. Particles having diameters smaller than about 0.5 microns can also be deposited in the alveolar region by sedimentation, although very small particles may be exhaled.

### B. Devices Used For Nasal And Pulmonary Drug Delivery

Drugs intended for intranasal delivery (systemic and local) can be administered as aqueous solutions or suspensions, as solutions or suspensions in halogenated hydrocarbon propellants (pressurized metered-dose inhalers), or as dry powders. Metered-dose spray pumps for aqueous formulations, pMDIs, and DPIs for nasal delivery are available from, for example, Valois of America or Pfeiffer of America.

Drugs intended for pulmonary delivery can also be administered as aqueous formulations, as suspensions or solutions in halogenated hydrocarbon propellants, or as dry powders. Aqueous formulations must be aerosolized by liquid nebulizers employing either hydraulic or ultrasonic atomization, propellant-based systems require suitable pressurized metered-dose inhalers (pMDIs), and dry powders require dry powder inhaler devices (DPIs) which are capable of dispersing the drug substance effectively. For aqueous and other non-pressurized liquid systems, a variety of nebulizers (including small volume nebulizers) are available to aerosolize the formulations. Compressor-driven nebulizers incorporate jet technology and use compressed air to generate the liquid aerosol. Such devices are commercially available from, for example, Healthdyne Technologies, Inc.; Invacare, Inc.; Mountain Medical Equipment, Inc.; Pari Respiratory, Inc.; Mada Medical, Inc.; Puritan-Bennet; Schuco, Inc., DeVilbiss Health Care, Inc.; and Hospitak, Inc. Ultrasonic nebulizers rely on mechanical energy in the form of vibration of a piezoelectric crystal to generate inhalable liquid droplets and are commercially available from, for example, Omron Heathcare, Inc. and DeVilbiss Health Care, Inc.

A propellant driven inhaler (pMDI) releases a metered dose of medicine upon each actuation. The medicine is formulated as a suspension or solution of a drug substance in a suitable propellant such as a halogenated hydrocarbon. pMDIs are described in, for example, Newman, S. P., Aerosols and the Lung, Clarke et al., eds., pp. 197-224 (Butterworths, London, England, 1984).

Dry powder inhalers (DPIs), which involve deaggregation and aerosolization of dry powders, normally rely upon a burst of inspired air that is drawn through the unit to deliver a drug dosage. Such devices are described in, for example, U.S. Pat. No. 4,807,814 to Douche et al., which is directed to a pneumatic powder ejector having a suction stage and an injection stage; SU 628930 (Abstract), describing a hand-held powder disperser having an axial air flow tube; Fox et al., Powder and Bulk Engineering, pages 33-36 (March 1988), describing a venturi eductor having an axial air inlet tube upstream of a venturi restriction; EP 347 779, describing a hand-held powder disperser having a collapsible expansion chamber, and U.S. Pat. No. 5,785,049 to Smith et al., directed to dry powder delivery devices for drugs.

### C. Problems With Conventional Aerosol And Injectable Compositions And Methods

Conventional techniques are extremely inefficient in delivering agents to the lung for a variety of reasons. Prior to the present invention, attempts to develop inhalable aqueous suspensions of poorly water soluble drugs have been largely unsuccessful. For example, it has been reported that ultrasonic nebulization of a suspension containing fluorescein and latex drug spheres, representing insoluble drug particles, resulted in only 1% aerosolization of the particles, while air jet nebulization resulted in only a fraction of particles being aerosolized (Susan L. Tiano, "Functionality Testing Used to Rationally Assess Performance of a Model Respiratory Solution or Suspension in a Nebulizer," Dissertation Abstracts International, 56/12-B, pp. 6578 (1995)). Another problem encountered with nebulization of liquid formulations prior to the present invention was the long (4-20 min) period of time required for administration of a therapeutic dose. Long administration times are required because conventional liquid formulations for nebulization are very dilute solutions or suspensions of micronized drug substance. Prolonged administration times are undesirable because they lessen patient compliance and make it difficult to control the dose administered. Lastly, aerosol formulations of micronized drug are not feasible for deep lung delivery of insoluble compounds because the droplets needed to reach the alveolar region (0.5 to 2 microns) are too small to accommodate micronized drug crystals, which are typically 2-3 microns or more in diameter.

Conventional pMDIs are also inefficient in delivering drug substance to the lung. In most cases, pMDIs consist of suspensions of micronized drug substance in halogenated hydrocarbons such as chlorofluorocarbons (CFCs) or hydrofluoroalkanes (HFAs). Actuation of the pMDI results in delivery of a metered dose of drug and propellant, both of which exit the device at high velocities because of the propellant pressures. The high velocity and momentum of the drug particles results in a high degree of oropharyngeal impaction as well as loss to the device used to deliver the agent. These losses lead to variability in therapeutic agent levels and poor therapeutic control. In addition, oropharyngeal deposition of drugs intended for topical administration to the conducting airways (such as corticosteroids) can lead to systemic absorption with resultant undesirable side effects. Additionally, conventional micronization (air jet milling) of pure drug substance can reduce the drug particle size to no less than about 2-3 microns. Thus, the micronized material typically used in pMDIs is inherently unsuitable for delivery to the alveolar region and is not expected to deposit below the central bronchiole region of the lung.

Prior to the present invention, delivery of dry powders to the lung typically used micronized drug substance. In the dry powder form, micronized substances tend to have substantial interparticle electrostatic attractive forces which prevent the powders from flowing smoothly and generally make them difficult to disperse. Thus, two key challenges to pulmonary delivery of dry powders are the ability of the device to accurately meter the intended dose and the ability of the device to fully disperse the micronized particles. For many devices and formulations, the extent of dispersion is dependent upon the patient's inspiration rate, which itself may be variable and can lead to a variability in the delivered dose.

Delivery of drugs to the nasal mucosa can also be accomplished with aqueous, propellant-based, or dry powder formulations. However, absorption of poorly soluble drugs can be problematic because of mucociliary clearance which transports deposited particles from the nasal mucosa to the throat where they are swallowed. Complete clearance generally occurs within about 15-20 minutes. Thus, poorly soluble drugs which do not dissolve within this time frame are unavailable for either local or systemic activity.

As described below in the Background of Nanoparticulate Active Agent Compositions, several published U.S. patents and patent applications describe aerosols of nanoparticulate drugs. However, none of these documents describe aerosols of a nanoparticulate benzodiazepine, such as lorazepam.

### II. Background Regarding Lorazepam

Lorazepam is a benzodiazepine. It is also known as 7-Chloro-5-(2-chloxophenyl)-1,3-dihydro-3-hydroxy-2*H*-1,4-benzodiazepin-2-one. Its molecular formula is C₁₅H₁₀ClN₂O₂, and it has a molecular weight of 321.16. Lorazepam has only slight solubility in water, *i.e.,* 0.08 mg/mL. United States Patent No. 6,699,849 to Loftsson et al., which is specifically incorporated by reference, refers to lorazepam and benzodiazepine. Lorazepam is a controlled substance. Merck Index, Thirteenth Ed., p. 999 (Merck & Co., Whitehouse Station, N.J. 2001). As pharmaceutically acceptable salts including organic salts or esters of lorazepam can be employed as a substitute for lorazepam, the references below to lorazepam are also intended to include lorazepam salts and esters and mixtures thereof.

Because of lorazepam's low water solubility, it is generally formulated for oral administration. However, oral administration of lorazepam has disadvantages. For example, lorazepam is susceptible to enzymatic degradation by glucuronyl transferase enzyme in the intestine or in the intestinal mucosa, as disclosed in United States Patent No. 6,692,766 to Rubinstein et al., which is incorporated by reference. Sterile lorazepam typically includes a preservative such as benzyl alcohol and requires refrigeration. Lorazepam delivered orally may have a slow absorption and onset of action.

Injectable formulations of lorazepam are preferable over oral administration doses because intravenous (IV) or intramuscular (IM) administration of a drug results in a significantly shorter response time as compared to oral administration. Moreover, injectable formulations of pain medication are also preferable for post-operative health care, where oral administration may not be feasible. Injectable formulations of lorazepam are particularly preferred, as lorazepam is not addictive, in contrast to other injectable formulations of drugs, such as morphine and ketorolac (Toradol^{®}).

However, injectable lorazepam formulations are difficult to formulate due to the low water-solubility of lorazepam. Moreover, current injectable formulations of lorazepam are undesirable because the formulations must include polyethylene glycol and propylene glycol as solubilizers, which can result in pain at the injection site.

### III. Background Regarding Nanoparticulate Active Agent Compositions

Nanoparticulate compositions, first described in U.S. Pat. No. 5,145,684 ("the'684 patent"), are particles consisting of a poorly soluble therapeutic or diagnostic agent having adsorbed onto or associated with the surface thereof a non-crosslinked surface stabilizer. The '684 patent also describes methods of making such nanoparticulate compositions but does not describe compositions comprising a benzodiazepine, such as lorazepam, in nanoparticulate form. Methods of making nanoparticulate compositions are described, for example, in U.S. Pat. Nos. 5,518,187 and 5,862,999, both for "Method of Grinding Pharmaceutical Substances;" U.S. Pat. No. 5,718,388, for "Continuous Method of Grinding Pharmaceutical Substances;" and U.S. Pat. No. 5,510,118 for "Process of Preparing Therapeutic Compositions Containing Nanoparticles."

Nanoparticulate compositions are also described, for example, in U.S. Pat. No. 5,298,262 for "Use of Ionic Cloud Point Modifiers to Prevent Particle Aggregation During Sterilization;" U.S. Pat. No. 5,302,401 for "Method to Reduce Particle Size Growth During Lyophilization;" U.S. Pat. No. 5,318,767 for "X-Ray Contrast Compositions Useful in Medical Imaging;" U.S. Pat. No. 5,326,552 for "Novel Formulation For Nanoparticulate X-Ray Blood Pool Contrast Agents Using High Molecular Weight Non-ionic Surfactants;" U.S. Pat. No. 5,328,404 for "Method of X-Ray Imaging Using Iodinated Aromatic Propanedioates;" U.S. Pat. No. 5,336,507 for "Use of Charged Phospholipids to Reduce Nanoparticle Aggregation;" U.S. Pat. No. 5,340,564 for "Formulations Comprising Olin 10-G to Prevent Particle Aggregation and Increase Stability;" U.S. Pat. No. 5,346,702 for "Use of Non-Ionic Cloud Point Modifiers to Minimize Nanoparticulate Aggregation During Sterilization;" U.S. Pat. No. 5,349,957 for "Preparation and Magnetic Properties of Very Small Magnetic-Dextran Particles;" U.S. Pat. No. 5,352,459 for "Use of Purified Surface Modifiers to Prevent Particle Aggregation During Sterilization;" U.S. Pat. Nos. 5,399,363 and 5,494,683, both for "Surface Modified Anticancer Nanoparticles;" U.S. Pat. No. 5,401,492 for "Water Insoluble Non-Magnetic Manganese Particles as Magnetic Resonance Enhancement Agents;" U.S. Pat. No. 5,429,824 for "Use of Tyloxapol as a Nanoparticulate Stabilizer;" U.S. Pat. No. 5,447,710 for "Method for Making Nanoparticulate X-Ray Blood Pool Contrast Agents Using High Molecular Weight Non-ionic Surfactants;" U.S. Pat. No. 5,451,393 for "X-Ray Contrast Compositions Useful in Medical Imaging;" U.S. Pat. No. 5,466,440 for "Formulations of Oral Gastrointestinal Diagnostic X-Ray Contrast Agents in Combination with Pharmaceutically Acceptable Clays;" U.S. Pat. No. 5,470,583 for "Method of Preparing Nanoparticle Compositions Containing Charged Phospholipids to Reduce Aggregation;" U.S. Pat. No. 5,472,683 for "Nanoparticulate Diagnostic Mixed Carbamic Anhydrides as X-Ray Contrast Agents for Blood Pool and Lymphatic System Imaging;" U.S. Pat. No. 5,500,204 for "Nanoparticulate Diagnostic Dimers as X-Ray Contrast Agents for Blood Pool and Lymphatic System Imaging;" U.S. Pat. No. 5,518,738 for "Nanoparticulate NSAID Formulations;" U.S. Pat. No. 5,521,218 for "Nanoparticulate Iododipamide Derivatives for Use as X-Ray Contrast Agents;" U.S. Pat. No. 5,525,328 for "Nanoparticulate Diagnostic Diatrizoxy Ester X-Ray Contrast Agents for Blood Pool and Lymphatic System Imaging;" U.S. Pat. No. 5,543,133 for "Process of Preparing X-Ray Contrast Compositions Containing Nanoparticles;" U.S. Pat. No. 5,552,160 for "Surface Modified NSAID Nanoparticles;" U.S. Pat. No. 5,560,931 for "Formulations of Compounds as Nanoparticulate Dispersions in Digestible Oils or Fatty Acids;" U.S. Pat. No. 5,565,188 for "Polyalkylene Block Copolymers as Surface Modifiers for Nanoparticles;" U.S. Pat. No. 5,569,448 for "Sulfated Non-ionic Block Copolymer Surfactant as Stabilizer Coatings for Nanoparticle Compositions;" U.S. Pat. No. 5,571,536 for "Formulations of Compounds as Nanoparticulate Dispersions in Digestible Oils or Fatty Acids;" U.S. Pat. No. 5,573,749 for "Nanoparticulate Diagnostic Mixed Carboxylic Anydrides as X-Ray Contrast Agents for Blood Pool and Lymphatic System Imaging;" U.S. Pat. No. 5,573,750 for "Diagnostic Imaging X-Ray Contrast Agents;" U.S. Pat. No. 5,573,783 for "Redispersible Nanoparticulate Film Matrices With Protective Overcoats;" U.S. Pat. No. 5,580,579 for "Site-specific Adhesion Within the GI Tract Using Nanoparticles Stabilized by High Molecular Weight, Linear Poly(ethylene Oxide) Polymers;" U.S. Pat. No. 5,585,108 for "Formulations of Oral Gastrointestinal Therapeutic Agents in Combination with Pharmaceutically Acceptable Clays;" U.S. Pat. No. 5,587,143 for "Butylene Oxide-Ethylene Oxide Block Copolymers Surfactants as Stabilizer Coatings for Nanoparticulate Compositions;" U.S. Pat. No. 5,591,456 for "Milled Naproxen with Hydroxypropyl Cellulose as Dispersion Stabilizer;" U.S. Pat. No. 5,593,657 for "Novel Barium Salt Formulations Stabilized by Non-ionic and Anionic Stabilizers;" U.S. Pat. No. 5,622,938 for "Sugar Based Surfactant for Nanocrystals;" U.S. Pat. No. 5,628,981 for "Improved Formulations of Oral Gastrointestinal Diagnostic X-Ray Contrast Agents and Oral Gastrointestinal Therapeutic Agents;" U.S. Pat. No. 5,643,552 for "Nanoparticulate Diagnostic Mixed Carbonic Anhydrides as X-Ray Contrast Agents for Blood Pool and Lymphatic System Imaging;" U.S. Pat. No. 5,718,388 for "Continuous Method of Grinding Pharmaceutical Substances;" U.S. Pat. No. 5,718,919 for "Nanoparticles Containing the R(-)Enantiomer of Ibuprofen;" U.S. Pat. No. 5,747,001 for "Aerosols Containing Beclomethasone Nanoparticle Dispersions;" U.S. Pat. No. 5,834,025 for "Reduction of Intravenously Administered Nanoparticulate Formulation Induced Adverse Physiological Reactions;" U.S. Pat. No. 6,045,829 "Nanocrystalline Formulations of Human Immunodeficiency Virus (HIV) Protease Inhibitors Using Cellulosic Surface Stabilizers;" U.S. Pat. No. 6,068,858 for "Methods of Making Nanocrystalline Formulations of Human Immunodeficiency Virus (HIV) Protease Inhibitors Using Cellulosic Surface Stabilizers;" U.S. Pat. No. 6,153,225 for "Injectable Formulations ofNanoparticulate Naproxen;" U.S. Pat. No. 6,165,506 for "New Solid Dose Form of Nanoparticulate Naproxen; U.S. Pat. No. 6,221,400 for "Methods of Treating Mammals Using Nanocrystalline Formulations of Human Immunodeficiency Virus (HIV) Protease Inhibitors;" U.S. Pat. No. 6,264,922 for "Nebulized Aerosols Containing Nanoparticle Dispersions;" U.S. Pat. No. 6,267,989 for "Methods for Preventing Crystal Growth and Particle Aggregation in Nanoparticle Compositions;" U.S. Pat. No. 6,270,806 for "Use of PEG-Derivatized Lipids as Surface Stabilizers for Nanoparticulate Compositions;" U.S. Pat. No. 6,316,029 for "Rapidly Disintegrating Solid Oral Dosage Form," U.S. Pat. No. 6,375,986 for "Solid Dose Nanoparticulate Compositions Comprising a Synergistic Combination of a Polymeric Surface Stabilizer and Dioctyl Sodium Sulfosuccinate;" U.S. Pat. No. 6,428,814 for "Bioadhesive Nanoparticulate Compositions Having Cationic Surface Stabilizers;" U.S. Pat. No. 6,431,478 for "Small Scale Mill;" U.S. Pat. No. 6,432,381 for "Methods for Targeting Drug Delivery to the Upper and/or Lower Gastrointestinal Tract;" U.S. Pat. No. 6,582,285 for "Apparatus for Sanitary Wet Milling;" and U.S. Pat. No. 6,592,903 for "Nanoparticulate Dispersions Comprising a Synergistic Combination of a Polymeric Surface Stabilizer and Dioctyl Sodium Sulfosuccinate;" 6,656,504 for "Nanoparticulate Compositions Comprising Amorphous Cyclosporine;" 6,742,734 for "System and Method for Milling Materials;" 6,745,962 for "Small Scale Mill and Method Thereof;" 6,811,767 for "Liquid droplet aerosols of nanoparticulate drugs;" and 6,908,626 for "Compositions having a combination of immediate release and controlled release characteristics;" all of which are specifically incorporated by reference. In addition, U.S. patent application Ser. No. 20020012675 A1, published on Jan. 31, 2002, for "Controlled Release Nanoparticulate Compositions" and WO 02/098565 for "System and Method for Milling Materials," describe nanoparticulate compositions, and are specifically incorporated by reference.

In particular, documents referring to aerosols of nanoparticulate drugs include U.S. Pat. No. 5,747,001 for "Aerosols Containing Beclomethasone Nanoparticle Dispersions" and U.S. Pat. No. 6,264,922 for "Nebulized Aerosols Containing Nanoparticle Dispersions," and documents referring to injectable compositions of nanoparticulate drugs include U.S. Pat. No. 6,153,225 for "Injectable Formulations of Nanoparticulate Naproxen," and U.S. Pat. Nos. 5,399,363 and 5,494,683, both for "Surface Modified Anticancer Nanoparticles." None of these documents describe injectable or aerosol compositions of a nanoparticulate benzodiazepine, such as lorazepam.

Amorphous small particle compositions are described, for example, in U.S. Pat. No. 4,783,484 for "Particulate Composition and Use Thereof as Antimicrobial Agent;" U.S. Pat. No. 4,826,689 for "Method for Making Uniformly Sized Particles from Water-Insoluble Organic Compounds;" U.S. Pat. No. 4,997,454 for "Method for Making Uniformly-Sized Particles From Insoluble Compounds;" U.S. Pat. No. 5,741,522 for "Ultrasmall, Non-aggregated Porous Particles of Uniform Size for Entrapping Gas Bubbles Within and Methods;" and U.S. Pat. No. 5,776,496, for "Ultrasmall Porous Particles for Enhancing Ultrasound Back Scatter" all of which are specifically incorporated herein by reference.

There remains a need in the art for improved dosage forms of benzodiazepines, such as lorazepam. The present invention satisfies this need.

### SUMMARY OF THE INVENTION

The present invention is directed to the surprising and unexpected discovery of new aerosol and injectable dosage forms of a nanoparticulate benzodiazepine, such as lorazepam. The formulations comprises a nanoparticulate benzodiazepine, such as nanoparticulate lorazepam, having an effective average particle size of less than about 2000 nm. The nanoparticulate benzodiazepine, such as lorazepam, preferably has at least one surface stabilizer either adsorbed onto or associated with the surface of the benzodizepine. In one embodiment of the invention, the surface stabilizer is a povidone polymer. Because lorazepam is practically insoluble in water, significant bioavailability can be problematic.

In one embodiment there is provided an aerosol that delivers an optimal dosage of a benzodiazepine, such as lorazepam. The aerosols of the invention do not require a preservative such as benzyl alcohol, which affects lorazepam stability.

In another embodiment, a safe and effective injectable formulation of a benzodiazepine, such as lorazepam, is provided. The injectable formulation eliminates the need for propylene glycol and polyethylene glycol, such as polyoxyl 60 hydrogenated castor oil (HCO-60), as solubilizers for injectable lorazepam compositions, and solves the problem of the insolubility of lorazepam in water. This is beneficial, as in convention non-nanoparticulate injectable benzodiazepine formulations comprising polyoxyl 60 hydrogenated castor oil as a solubilizer, the presence of this solubilizer can lead to anaphylactic shock (i.e., severe allergic reaction) and death. The injectable dosage forms of the invention surprisingly deliver the required therapeutic amount of the drug *in vivo,* and render the drug bioavailable in a rapid and constant manner, which is required for effective human therapy. Moreover, the invention provides for compositions comprising high concentrations of a benzodiazepine, such as lorazepam, in low injection volumes, with rapid drug dissolution upon administration.

The present invention is also directed to aqueous, propellant-based, and dry powder aerosols of a nanoparticulate benzodiazepine, such as lorazepam, for pulmonary and nasal delivery, in which essentially every inhaled particle contains at least one nanoparticulate benzodiazepine, such as lorazepam, nanoparticle. The nanoparticulate benzodiazepine, such as lorazepam, is highly water-insoluble. Preferably, the nanoparticulate benzodiazepine, such as lorazepam, has an effective average particle size of less than about 2 microns. Nanoparticulate aerosol formulations are described in U.S. Patent No. 6,811,767 to Bosch et al., specifically incorporated by reference. Non-aerosol preparations of submicron sized water-insoluble drugs are described in U.S. Pat. No. 5,145,684 to Liversidge et al., specifically incorporated herein by reference.

The invention also includes the following embodiments directed to aerosol formulations of a benzodiazepine, such as lorazepam. One embodiment of the invention is directed to aqueous aerosols of nanoparticulate dispersion of a benzodiazepine, such as lorazepam. Another embodiment of the invention is directed to dry powder aerosol formulations comprising a benzodiazepine, such as lorazepam, for pulmonary and/or nasal administration. Yet another embodiment of the invention is directed to a process and composition for propellant-based systems comprising a nanoparticulate benzodiazepine, such as lorazepam.

The nanoparticulate benzodiazepine, such as lorazepam, formulations of the invention may optionally include one or more pharmaceutically acceptable excipients, such as non-toxic physiologically acceptable liquid carriers, pH adjusting agents, or preservatives.

In another aspect of the invention there is provided a method of preparing the nanoparticulate benzodiazepine, such as lorazepam, injectable and aerosol formulations of the invention. The nanoparticulate dispersions used in making aerosol and injectable nanoparticulate benzodiazepine compositions can be made by wet milling, homogenization, precipitation, or supercritical fluid methods known in the art. An exemplary method comprises: (1) dispersing a benzodiazepine, such as lorazepam, in a liquid dispersion media; and (2) mechanically reducing the particle size of the benzodiazepine to the desired effective average particle size, e.g., less than about 2000 nm. At least one surface stabilizer can be added to the dispersion media either before, during, or after particle size reduction of the benzodiazepine. In one embodiment for the injectable composition, the surface stabilizer is a povidone polymer with a molecular weight of less than about 40,000 daltons. Preferably, the liquid dispersion media is maintained at a physiologic pH, for example, within the range of from about 3 to about 8, during the size reduction process. The nanoparticulate benzodiazepine dispersion can be used as an injectable formulation.

Dry powders comprising a nanoparticulate benzodiazepine, such as lorazepam, can be made by spray drying or freeze-drying aqueous dispersions of the nanoparticles. The dispersions used in these systems may or may not comprise dissolved diluent material prior to drying. Additionally, both pressurized and non-pressurized milling operations can be employed to make nanoparticulate benzodiazepine, such as lorazepam, compositions in non-aqueous systems.

In yet another aspect of the invention, there is provided a method of treating a subject in need with the injectable and/or aerosol nanoparticulate benzodiazepine, such as lorazepam, compositions of the invention. In an exemplary method, therapeutically effective amount of an injectable or aerosol nanoparticulate benzodiazepine composition of the invention is administered to a subject in need. The methods of the invention encompass treating a-subject for status epilepticus, treatment of irritable bowel syndrome, sleep induction, acute psychosis, and pre-anesthesia medication. Diagnostic methods, comprising imaging of the administered dosage form, are also encompassed by the invention.

It is to be understood that both the foregoing general description and the following detailed description are exemplary and explanatory and are intended to provide further explanation of the invention as claimed. Other objects, advantages, and novel features will be readily apparent to those skilled in the art from the following detailed description of the invention.

### DETAILED DESCRIPTION OF THE INVENTION

The compositions of the invention encompass a nanoparticulate benzodiazepine, such as lorazepam, having an effective average particle size of less than about 2000 nm. For the injectable compositions, the nanoparticulate benzodiazepine, such as lorazepam, preferably has an effective average particle size of less than about 600 nm. For the aerosol compositions, the nanoparticulate benzodiazepine, such as lorazepam, has an effective average particle size of less than about 2000 nm. In one embodiment of the invention, the nanoparticulate benzodiazepine particles have at least one surface stabilizer either adsorbed onto or associated with the surface of the drug particles. The compositions are formulated into either an aerosol dosage form or an injectable dosage form. The aerosol dosage form can be either an aqueous aerosol or a dry powder aerosol.

Using the nanoparticulate benzodiazepine aerosol compositions of the invention, an essentially water-insoluble benzodiazepine, such as lorazepam, can be delivered to the deep lung. This is either not possible or extremely difficult using aerosol formulations of a micronized water-insoluble benzodiazepine. Deep lung delivery is necessary for benzodiazepine, such as lorazepam, compositions that are intended for systemic administration because deep lung delivery allows rapid absorption of the drug into the bloodstream by the alveoli, thus enabling rapid onset of action.

The present invention increases the number of benzodiazepine, such as lorazepam, particles per unit dose and results in distribution of a nanoparticulate benzodiazepine, such as lorazepam, over a larger physiological surface area as compared to the same quantity of a delivered micronized benzodiazepine, such as lorazepam. For systemic delivery by the pulmonary route, this approach takes maximum advantage of the extensive surface area presented in the alveolar region — thus producing more favorable benzodiazepine, such as lorazepam, delivery profiles, such as a more complete absorption and rapid onset of action.

Moreover, in contrast to micronized aqueous aerosol dispersions, aqueous dispersions of a water-insoluble nanoparticulate benzodiazepine, such as lorazepam, can be nebulized ultrasonically. Micronized drug is too large to be delivered efficiently by an ultrasonic nebulizer.

Droplet size determines *in vivo* deposition of a benzodiazepine, *i.e.,* very small particles, about <2 microns, are delivered to the alveoli; larger particles, about 2 to about 10 microns, are delivered to the bronchiole region; and for nasal delivery, particles of about 5 to about 100 microns are preferred. Thus, the ability to obtain very small benzodiazepine, such as lorazepam, particle sizes which can "fit" in a range of droplet sizes allows more effective and more efficient *(i. e.,* benzodiazepine uniformity) targeting to the desired delivery region. This is not possible using micronized benzodiazepine, as the particle size of benzodiazepine is too large to target areas such as the alveolar region of the lung. Moreover, even when micronized benzodiazepine is incorporated into larger droplet sizes, the resultant aerosol formulation is heterogeneous *(i.e.,* not all droplets contain benzodiazepine), and does not result in the rapid and efficient benzodiazepine delivery enabled by the nanoparticulate aerosol benzodiazepine, such as lorazepam, formulations of the invention.

The present invention also enables the aqueous aerosol delivery of high doses of benzodiazepine, such as lorazepam, in an extremely short time period, *i.e.,* 1-2 seconds (1 puff). This is in contrast to the conventional 4-20 min. administration period observed with pulmonary aerosol formulations of micronized drug. Furthermore, the dry aerosol nanoparticulate benzodiazepine, such as lorazepam, powders of the present invention are spherical and can be made smaller than micronized material, thereby producing aerosol compositions having better flow and dispersion properties, and capable of being delivered to the deep lung.

Finally, the aerosol benzodiazepine, such as lorazepam, compositions of the present invention enable rapid nasal delivery. Nasal delivery of such aerosol compositions will be absorbed more rapidly and completely than micronized aerosol compositions before being cleared by the mucociliary mechanism.

The dosage forms of the present invention may be provided in formulations which exhibit a variety of release profiles upon administration to a patient including, for example, an IR formulation, a CR formulation that allows once per day administration, and a combination of both IR and CR formulations. Because CR forms of the present invention can require only one dose per day (or one dose per suitable time period, such as weekly or monthly), such dosage forms provide the benefits of enhanced patient convenience and compliance. The mechanism of controlled-release employed in the CR form may be accomplished in a variety of ways including, but not limited to, the use of erodable formulations, diffusion-controlled formulations, and osmotically-controlled formulations.

Advantages of the nanoparticulate benzodiazepine formulations of the invention over conventional forms of a benzodiazepine, such as lorazepam (e.g., non-nanoparticulate or solubilized dosage forms) include, but are not limited to: (1) increased water solubility; (2) increased bioavailability; (3) smaller dosage form size due to enhanced bioavailability; (4) lower therapeutic dosages due to enhanced bioavailability; (5) reduced risk of unwanted side effects due to lower dosing; and (6) enhanced patient convenience and compliance. A further advantage of the injectable nanoparticulate benzodiazepine formulation of the present invention over conventional forms of injectable benzodiazepines, such as lorazepam, is the elimination of the need to use polyoxyl 60 hydrogenated castor oil (HCO-60) as a solubilizer. A further advantage of the aerosol nanoparticulate benzodiazepines, such as lorazepam, is a reduced risk of unwanted side effects.

The present invention also includes nanoparticulate benzodiazepine, such as lorazepam, compositions, together with one or more non-toxic physiologically acceptable carriers, adjuvants, or vehicles, collectively referred to as carriers. The compositions can be formulated for parenteral injection (e.g., intravenous, intramuscular, or subcutaneous) or aerosol delivery. The aerosols can be used for any suitable delivery, such as pulmonary or nasal delivery.

The present invention is described herein using several definitions, as set forth below and throughout the application.

The term "effective average particle size of less than about 2000 nm", as used herein means that at least 50% of the benzodiazepine, such as lorazepam, particles have a size, by weight, of less than about 2000 nm, when measured by, for example, sedimentation field flow fractionation, photon correlation spectroscopy, light scattering, disk centrifugation, and other techniques known to those of skill in the art.

As used herein, "about" will be understood by persons of ordinary skill in the art and will vary to some extent on the context in which it is used. If there are uses of the term which are not clear to persons of ordinary skill in the art given the context in which it is used, "about" will mean up to plus or minus 10% of the particular term.

As used herein with reference to a stable benzodiazepine, such as lorazepam, particle connotes, but is not limited to one or more of the following parameters: (1) benzodiazepine particles do not appreciably flocculate or agglomerate due to interparticle attractive forces or otherwise significantly increase in particle size over time; (2) that the physical structure of the benzodiazepine particles is not altered over time, such as by conversion from an amorphous phase to a crystalline phase; (3) that the benzodiazepine particles are chemically stable; and/or (4) where the benzodiazepine has not been subject to a heating step at or above the melting point of the benzodiazepine in the preparation of the nanoparticles of the present invention.

The term "conventional" or "non-nanoparticulate" active agent or benzodiazepine, such as lorazepam, shall mean an active agent, such as lorazepam, which is solubilized or which has an effective average particle size of greater than about 2000 nm. Nanoparticulate active agents as defined herein have an effective average particle size of less than about 2000 nm.

The phrase "poorly water soluble drugs" as used herein refers to those drugs that have a solubility in water of less than about 30 mg/ml, preferably less than about 20 mg/ml, preferably less than about 10 mg/ml, or preferably less than about 1 mg/ml.

As used herein, the phrase "therapeutically effective amount" shall mean that drug dosage that provides the specific pharmacological response for which the drug is administered in a significant number of subjects in need of such treatment. It is emphasized that a therapeutically effective amount of a drug that is administered to a particular subject in a particular instance will not always be effective in treating the conditions/diseases described herein, even though such dosage is deemed to be a therapeutically effective amount by those of skill in the art.

The term "particulate" as used herein refers to a state of matter which is characterized by the presence of discrete particles, pellets, beads or granules irrespective of their size, shape or morphology. The term "multiparticulate" as used herein means a plurality of discrete, or aggregated, particles, pellets, beads, granules or mixture thereof irrespective of their size, shape or morphology.

The term "modified release" as used herein in relation to the composition according to the invention means release which is not immediate release and is taken to encompass controlled release, sustained release, and delayed release.

The term "time delay" as used herein refers to the duration of time between administration of the composition and the release of benzodiazepine, such as lorazepam, from a particular component.

The term "lag time" as used herein refers to the time between delivery of active ingredient from one component and the subsequent delivery of benzodiazepine, such as lorazepam, from another component.

### I. Preferred Characteristics of the Nanoparticulate Benzodiazepine Compositions

There are a number of enhanced pharmacological characteristics of the nanoparticulate benzodiazepine, such as lorazepam, compositions of the present invention.

### A. Increased Bioavailability

The benzodiazepine, such as lorazepam, formulations of the present invention exhibit increased bioavailability at the same dose of the same benzodiazepine, such as lorazepam, and require smaller doses as compared to prior conventional benzodiazepine, such as lorazepam, formulations.

Moreover, a nanoparticulate benzodiazepine, such as lorazepam, dosage form requires less drug to obtain the same pharmacological effect observed with a conventional microcrystalline benzodiazepine, such as lorazepam, dosage form. Therefore, the nanoparticulate benzodiazepine, such as lorazepam, dosage form has an increased bioavailability as compared to the conventional microcrystalline benzodiazepine, such as lorazepam, dosage form.

### B. The Pharmacokinetic Profiles of the Benzodiazepine Compositions of the Invention are not Affected by the Fed or Fasted State of the Subject Ingesting the Compositions

The compositions of the present invention encompass a benzodiazepine, such as lorazepam, wherein the pharmacokinetic profile of the benzodiazepine is not substantially affected by the fed or fasted state of a subject ingesting the composition. This means that there is little or no appreciable difference in the quantity of drug absorbed or the rate of drug absorption when the nanoparticulate benzodiazepine, such as lorazepam, compositions are administered in the fed versus the fasted state.

Benefits of a dosage form which substantially eliminates the effect of food include an increase in subject convenience, thereby increasing subject compliance, as the subject does not need to ensure that they are taking a dose either with or without food. This is significant, as with poor subject compliance with a benzodiazepine, such as lorazepam" an increase in the medical condition for which the drug is being prescribed may be observed.

The invention also preferably provides a benzodiazepine, such as lorazepam, compositions having a desirable pharmacokinetic profile when administered to mammalian subjects. The desirable pharmacokinetic profile of the benzodiazepine, such as lorazepam, compositions preferably includes, but is not limited to: (1) a Cₘₐₓ for benzodiazepine, when assayed in the plasma of a mammalian subject following administration, that is preferably greater than the Cₘₐₓ for a non-nanoparticulate benzodiazepine formulation administered at the same dosage; and/or (2) an AUC for benzodiazepine, when assayed in the plasma of a mammalian subject following administration, that is preferably greater than the AUC for a non-nanoparticulate benzodiazepine formulation, administered at the same dosage; and/or (3) a Tmax for benzodiazepine, when assayed in the plasma of a mammalian subject following administration, that is preferably less than the Tmax for a non-nanoparticulate benzodiazepine formulation, administered at the same dosage. The desirable pharmacokinetic profile, as used herein, is the pharmacokinetic profile measured after the initial dose of the benzodiazepine.

In one embodiment, a preferred benzodiazepine, such as lorazepam, composition exhibits in comparative pharmacokinetic testing with a non-nanoparticulate benzodiazepine, such as lorazepam, formulation, administered at the same dosage, a Tₘₐₓ not greater than about 90%, not greater than about 80%, not greater than about 70%, not greater than about 60%, not greater than about 50%, not greater than about 30%, not greater than about 25%, not greater than about 20%, not greater than about 15%, not greater than about 10%, or not greater than about 5% of the Tₘₐₓ exhibited by the non-nanoparticulate benzodiazepine, such as lorazepam, formulation.

In another embodiment, the benzodiazepine, such as lorazepam, composition of the invention exhibits in comparative pharmacokinetic testing with a non-nanoparticulate benzodiazepine, such as lorazepam, formulation, administered at the same dosage, a Cₘₐₓ which is at least about 50%, at least about 100%, at least about 200%, at least about 300%, at least about 400%, at least about 500%, at least about 600%, at least about 700%, at least about 800%, at least about 900%, at least about 1000%, at least about 1100%, at least about 1200%, at least about 1300%, at least about 1400%, at least about 1500%, at least about 1600%, at least about 1700%, at least about 1800%, or at least about 1900% greater than the Cₘₐₓ exhibited by the non-nanoparticulate benzodiazepine, such as lorazepam, formulation.

In yet another embodiment, the benzodiazepine, such as lorazepam, composition of the invention exhibits in comparative pharmacokinetic testing with a non-nanoparticulate benzodiazepine, such as lorazepam, formulation, administered at the same dosage, an AUC which is at least about 25%, at least about 50%, at least about 75%, at least about 100%, at least about 125%, at least about 150%, at least about 175%, at least about 200%, at least about 225%, at least about 250%, at least about 275%, at least about 300%, at least about 350%, at least about 400%, at least about 450%, at least about 500%, at least about 550%, at least about 600%, at least about 750%, at least about 700%, at least about 750%, at least about 800%, at least about 850%, at least about 900%, at least about 950%, at least about 1000%, at least about 1050%, at least about 1100%, at least about 1150%, or at least about 1200% greater than the AUC exhibited by the non-nanoparticulate benzodiazepine, such as lorazepam, formulation.

### C. Bioequivalency of the Benzodiazepine Compositions of the Invention When Administered in the Fed Versus the Fasted State

The invention also encompasses a composition comprising a nanoparticulate benzodiazepine, such as lorazepam, in which administration of the composition to a subject in a fasted state is bioequivalent to administration of the composition to a subject in a fed state.

The difference in absorption of the compositions comprising the nanoparticulate benzodiazepine, such as lorazepam, when administered in the fed versus the fasted state, is preferably less than about 100%, less than about 90%, less than about 80%, less than about 70%, less than about 60%, less than about 50%, less than about 40%, less than about 35%, less than about 30%, less than about 25%, less than about 20%, less than about 15%, less than about 10%, less than about 5%, or less than about 3%.

In one embodiment of the invention, the invention encompasses nanoparticulate benzodiazepine, such as lorazepam, wherein administration of the composition to a subject in a fasted state is bioequivalent to administration of the composition to a subject in a fed state, in particular as defined by Cₘₐₓ and AUC guidelines given by the U.S. Food and Drug Administration and the corresponding European regulatory agency (EMEA). Under U.S. FDA guidelines, two products or methods are bioequivalent if the 90% Confidence Intervals (CI) for AUC and Cₘₐₓ are between 0.80 to 1.25 (Tₘₐₓ measurements are not relevant to bioequivalence for regulatory purposes). To show bioequivalency between two compounds or administration conditions pursuant to Europe's EMEA guidelines, the 90% Cl for AUC must be between 0.80 to 1.25 and the 90% CI for Cₘₐₓ must between 0.70 to 1.43.

### D. Dissolution Profiles of the Benzodiazepine Compositions of the Invention

The benzodiazepine, such as lorazepam, compositions of the present invention have unexpectedly dramatic dissolution profiles. Rapid dissolution of an administered active agent is preferable, as faster dissolution generally leads to faster onset of action and greater bioavailability. To improve the dissolution profile and bioavailability of benzodiazepine, such as lorazepam" it is useful to increase the drug's dissolution so that it could attain a level close to 100%.

The benzodiazepine, such as lorazepam, compositions of the present invention preferably have a dissolution profile in which within about 5 minutes at least about 20% of the composition is dissolved. In other embodiments of the invention, at least about 30% or about 40% of the benzodiazepine, such as lorazepam, composition is dissolved within about 5 minutes. In yet other embodiments of the invention, preferably at least about 40%, about 50%, about 60%, about 70%, or about 80% of the benzodiazepine, such as lorazepam, composition is dissolved within about 10 minutes. Finally, in another embodiment of the invention, preferably at least about 70%, about 80%, about 90%, or about 100% of the benzodiazepine, such as lorazepam, composition is dissolved within about 20 minutes.

Dissolution is preferably measured in a medium which is discriminating. Such a dissolution media will produce two very different dissolution curves for two products having very different dissolution profiles in gastric juices, *i.e.,* the dissolution medium is predictive of *in vivo* dissolution of a composition. An exemplary dissolution medium is an aqueous medium containing the surfactant sodium lauryl sulfate at 0.025 M. Determination of the amount dissolved can be carried out by spectrophotometry. The rotating blade method (European Pharmacopoeia) can be used to measure dissolution.

### E. Redispersibility Profiles of the Benzodiazepine Compositions of the Invention

An additional feature of the benzodiazepine, such as lorazepam, compositions of the present invention is that the compositions redisperse such that the effective average particle size of the redispersed benzodiazepine, such as lorazepam, particles is less than about 2 microns. This is significant, as if upon administration the nanoparticulate benzodiazepine, such as lorazepam, compositions of the invention did not redisperse to a nanoparticulate particle size, then the dosage form may lose the benefits afforded by formulating the benzodiazepine, such as lorazepam, into a nanoparticulate particle size. A nanoparticulate size suitable for the present invention is an effective average particle size of less than about 2000 nm. In another embodiment, a nanoparticulate size suitable for the present invention is an effective average particle size of less than about 600 nm

Indeed, the nanoparticulate active agent compositions of the present invention benefit from the small particle size of the active agent; if the active agent does not redisperse into a small particle size upon administration, then "clumps" or agglomerated active agent particles are formed, owing to the extremely high surface free energy of the nanoparticulate system and the thermodynamic driving force to achieve an overall reduction in free energy. With the formation of such agglomerated particles, the bioavailability of the dosage form may fall well below that observed with the liquid dispersion form of the nanoparticulate active agent.

Moreover, the nanoparticulate benzodiazepine, such as lorazepam, compositions of the invention exhibit dramatic redispersion of the nanoparticulate benzodiazepine, such as lorazepam, particles upon administration to a mammal, such as a human or animal, as demonstrated by reconstitution/redispersion in a biorelevant aqueous media such that the effective average particle size of the redispersed benzodiazepine, such as lorazepam, particles is less than about 2 microns. Such biorelevant aqueous media can be any aqueous media that exhibit the desired ionic strength and pH, which form the basis for the biorelevance of the media. The desired pH and ionic strength are those that are representative of physiological conditions found in the human body. Such biorelevant aqueous media can be, for example, aqueous electrolyte solutions or aqueous solutions of any salt, acid, or base, or a combination thereof, which exhibit the desired pH and ionic strength.

Biorelevant pH is well known in the art. For example, in the stomach, the pH ranges from slightly less than 2 (but typically greater than 1) up to 4 or 5. In the small intestine the pH can range from 4 to 6, and in the colon it can range from 6 to 8. Biorelevant ionic strength is also well known in the art. Fasted state gastric fluid has an ionic strength of about 0.1M while fasted state intestinal fluid has an ionic strength of about 0.14. *See e.g.,* Lindahl et al., "Characterization of Fluids from the Stomach and Proximal Jejunum in Men and Women," Pharm. Res., 14 (4): 497-502 (1997).

It is believed that the pH and ionic strength of the test solution is more critical than the specific chemical content. Accordingly, appropriate pH and ionic strength values can be obtained through numerous combinations of strong acids, strong bases, salts, single or multiple conjugate acid-base pairs (i.e., weak acids and corresponding salts of that acid), monoprotic and polyprotic electrolytes, etc.

Representative electrolyte solutions can be, but are not limited to, HCl solutions, ranging in concentration from about 0.001 to about 0.1 M, and NaCl solutions, ranging in concentration from about 0.001 to about 0.1 M, and mixtures thereof. For example, electrolyte solutions can be, but are not limited to, about 0.1 M HCl or less, about 0.01 M HCl or less, about 0.001 M HCl or less, about 0.1 M NaCl or less, about 0.01 M NaCl or less, about 0.001 M NaCl or less, and mixtures thereof. Of these electrolyte solutions, 0.01 M HCl and/or 0.1 M NaCl, are most representative of fasted human physiological conditions, owing to the pH and ionic strength conditions of the proximal gastrointestinal tract.

Electrolyte concentrations of 0.001 M HCl, 0.01 M HCl, and 0.1 M HCl correspond to pH 3, pH 2, and pH 1, respectively. Thus, a 0.01 M HCl solution simulates typical acidic conditions found in the stomach. A solution of 0.1 M NaCl provides a reasonable approximation of the ionic strength conditions found throughout the body, including the gastrointestinal fluids, although concentrations higher than 0.1 M may be employed to simulate fed conditions within the human GI tract.

Exemplary solutions of salts, acids, bases or combinations thereof, which exhibit the desired pH and ionic strength, include but are not limited to phosphoric acid/phosphate salts + sodium, potassium and calcium salts of chloride, acetic acid/acetate salts + sodium, potassium and calcium salts of chloride, carbonic acid/bicarbonate salts + sodium, potassium and calcium salts of chloride, and citric acid/citrate salts + sodium, potassium and calcium salts of chloride.

In other embodiments of the invention, the redispersed benzodiazepine, such as lorazepam, particles of the invention (redispersed in an aqueous, biorelevant, or any other suitable media) have an effective average particle size of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 650 nm, less than about 600 nm, less than about 550 nm, less than about 500 nm, less than about 450 nm, less than about 400 nm, less than about 350 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods. Such methods suitable for measuring effective average particle size are known to a person of ordinary skill in the art.

Redispersibility can be tested using any suitable means known in the art. *See e.g.,* the example sections of U.S. Patent No. 6,375,986 for "Solid Dose Nanoparticulate Compositions Comprising a Synergistic Combination of a Polymeric Surface Stabilizer and Dioctyl Sodium Sulfosuccinate."

### F. Benzodiazepine Compositions Used in Conjunction with Other Active Agents

The benzodiazepine, such as lorazepam, compositions of the invention can additionally comprise one or more compounds useful in the condition to be treated. Examples of such other active agents include, but are not limited to, antidepressants, steroids, antiemetics, antinauseants, spasmolytics, antipsychotics, opioids, carbidopa/levodopa or dopamine agonists, anesthetics, and narcotics.

Examples of antidepressants include, but are not limited to, selective serotonin reuptake inhibitors (SSRIs) and tricyclic antidepressants (tricyclics). SSRIs include drugs such as escitalopram (brand name: Lexapro) citalopram (brand name: Celexa), fluoxetine (brand name: Prozac), paroxetine (brand name: Paxil) and sertraline (brand name: Zoloft). Tricyclics include amitriptyline (brand name: Elavil), desipramine (brand name: Norpramin), imipramine (brand name: Tofranil) and nortriptyline (brand names: Aventyl, Pamelor). Other antidepressants exist that have different ways of working than the SSRIs and tricylics. Commonly used ones are venlafaxine (brand name: Effexor), nefazadone (brand name: Serzone), bupropion (brand name: Wellbutrin), mirtazapine (brand name: Remeron) and trazodone (brand name: Desyrel). Less commonly used are the monomine oxidase inhibitors (MAOIs), such as phenelzine (brand name: Nardil) and tranylcypromine (brand name: Parnate).

Examples of steroids include, but are not limited to, betamethasone, budesonide, cortisone, dexamethasone, hydrocortisone, methylprednisolone, prednisolone, prednisone, and triamcinolone.

Examples of antiemetics or antinauseants include, but are not limited to, promethazine (Phenergan®), metoclopramide (Reglan®), cyclizine (Merezine®), diphenhydramine (Benadryl®), meclizine (Antivert®, Bonine®), chlorpromazine (Thorazine®), droperidol (Inapsine®), hydroxyzine (Atarax®, Vistaril®), prochlorperazine (Compazine®), trimethobenzamide (Tigan®), cisapride; h2-receptor antagonists, such as nizatidine, ondansetron (Zofran®), corticosteriods, 5-Hydroxytryptamine antagonists, such as dolasetron (Anzemet®), granisetron (Kytril®), ondansetron (Zofran®), tropisetron; dopamine antagonists, such as domperidone (Motilium®), droperidol (Inapsine®), haloperidol (Haldol®), chlorpromazine (Thorazine®); Antihistamines (5HT2 receptor antagonists), such as cyclizine (Antivert®, Bonine®, Dramamine®, Marezine®, Meclicot®, Medivert®), diphenhydramine, dimenhydrinate (Alavert®, Allegra®, Dramanate®) dimenhydrinate (Driminate®); and cannabinoids, such as marijuana and marinol.

Examples of spasmolytics or antispasmodics include, but are not limited to, methocarbamol, guaifenesin, diazepam, dantrolene, phenytoin, tolterodine, oxybutynin, flavoxate, and emepronium.

Examples of antipsychotics include, but are not limited to, clozapine (Clozaril®), risperidone (Risperdal®), olanzapine (Zyprexa®), quetiapine (Seroquel®), ziprasidone (Geodon®), and aripiprazole (Abilify®).

Examples of opioids include, but are not limited to, (1) opium alkaloids, such as morphine (Kadian®, Avinza®), codeine, and thebaine; (2) semisynthetic opioid derivatives, such as diamorphine (heroin), oxycodone (OxyContin®, Percodan®, Percocet®), hydrocodone, dihydrocodeine, hydromorphine, oxymorphone, and nicomorphine; (3) synthetic opioids, such as (a) pheylheptylamines, including methadone and levo-alphacetylmethadol (LAAM), (b) phenylpiperidines, including pethidine (meperidine), fentanyl, alfentanil, sufentanil, remifentanil, ketobemidone, and carfentanyl, (c) diphenylpropylamine derivatives, such as propoxyphene, dextropropoxyphene, dextromoramide, bezitramide, and piritramide, (d) benzomorphan derivatives, such as pentazocine and phenzocine, (e) oripavine derivatives, such as buprenorphine, (f) morphinan derivatives, such as butorphanol and nalbufine, and miscellaneous other synthetic opioids, such as dezocine, etorphine, tilidine, tramadol, loperamide, and diphenoxylate (Lomotil®).

Examples of carbidopa/levodopa or dopamine agonists include, but are not limited to, ropinirole, pramipexole and cabergoline, bromocriptine mesylate (Parlodel®), pergolide mesylate (Permax®), pramipexole dihydrochloride (Mirapex®), and ropinirole hydrochloride (Requip)™.

Examples of anesthetics include, but are not limited to, enflurane, halothane, isoflurane, methoxyflurane, nitrous oxide, etomidate, ketamine, methohexital, propofol, and thiopental.

### II. Compositions

The invention provides compositions comprising nanoparticulate benzodiazepine, such as lorazepam, particles and at least one surface stabilizer. The surface stabilizers are preferably adsorbed to or associated with the surface of the benzodiazepine, such as lorazepam, particles. Surface stabilizers useful herein do not chemically react with the benzodiazepine, such as lorazepam, particles or itself. Preferably, individual molecules of the surface stabilizer are essentially free of intermolecular cross-linkages. In another embodiment, the compositions of the present invention can comprise two or more surface stabilizers.

The present invention also includes nanoparticulate benzodiazepine, such as lorazepam, compositions together with one or more non-toxic physiologically acceptable carriers, adjuvants, or vehicles, collectively referred to as carriers. The compositions can be formulated for parenteral injection *(e.g.,* intravenous, intramuscular, or subcutaneous) or aerosol delivery. In certain embodiments of the invention, the nanoparticulate benzodiazepine, such as lorazepam, formulations are in an injectable form or an aerosol dosage form.

### A. Benzodiazepine Particles

The invention is practiced with a benzodiazepine, such as lorazepam. The benzodiazepine, such as lorazepam, is preferably present in an essentially pure form, is poorly soluble, and is dispersible in at least one liquid media. By "poorly soluble," it is meant that the benzodiazepine, such as lorazepam, has a solubility in the liquid dispersion media of less than about 10 mg/mL, and preferably of less than about 1 mg/mL. As noted above, the solubility of lorazepam in water is 0.08 mg/mL.

The drug can be selected from a variety of benzodiazepines for treatment of status epilepticus, treatment of irritable bowel syndrome, sleep induction, acute psychosis, and pre-anesthesia medications. Preferable drug classes are benzodiazepine, such as lorazepam, and pharmaceutically acceptable salts and esters of lorazepam. Benzodiazepines of particular interest are alprazolam, brotizolam, chlordiazepoxide, clobazam, clonazepam, clorazepam, demoxazepam, flumazenil, flurazepam halazepam, midazolam, nordazepam, medazepam, diazepam, nitrazepam oxazepam, midazepam, lorazepam, prazepam, quazepam, triazolam, temazepam, and loprazolam. Particularly preferred benzodiazepines are alprazolam, midazolam, clonazepam, lorazepam, and triazolam. The preferred benzodiazepine is lorazepam. A description of these classes of benzodiazepines and a listing of species within each class can be found in Martindale, The Extra Pharmacopoeia, Twenty-ninth Edition (The Pharmaceutical Press, London, 1989), specifically incorporated by reference. The drugs are commercially available and/or can be prepared by techniques known in the art.

"Pharmaceutically acceptable" as used herein refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

"Pharmaceutically acceptable salts and esters" as used herein refers to derivatives wherein the benzediazepine, such as lorazepam, is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quarternary ammonium salts of the benzodiazepine and preferably, lorazepam formed, for example, from non-toxic inorganic or organic acids. For example, such conventional non-toxic salts include those derived from inorganic acids such as hydrochloric, hydrobromic, sulfuric, sulfamic, phosphoric, nitric, and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

### B. Surface Stabilizers

Suitable surface stabilizers can be selected from known organic and inorganic pharmaceutical excipients. Such excipients include various polymers, low molecular weight oligomers, natural products, and surfactants. Preferred surface stabilizers include nonionic, ionic, cationic, anionic, and zwitterionic surfactants. A preferred surface stabilizer for an injectable nanoparticulate benzodiazepine formulation is a povidone polymer. Two or more surface stabilizers can be used in combination.

Representative examples of surface stabilizers include hydroxypropyl methylcellulose (now known as hypromellose), hydroxypropylcellulose, polyvinylpyrrolidone, sodium lauryl sulfate, dioctylsulfosuccinate, gelatin, casein, lecithin (phosphatides), dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers (e.g., macrogol ethers such as cetomacrogol 1000), polyoxyethylene castor oil derivatives, polyoxyethylene sorbitan fatty acid esters (e.g., the commercially available Tweens® such as e.g., Tween 20® and Tween 80® (ICI Speciality Chemicals)); polyethylene glycols (e.g., Carbowaxes 3550® and 934® (Union Carbide)), polyoxyethylene stearates, colloidal silicon dioxide, phosphates, carboxymethylcellulose calcium, carboxymethylcellulose sodium, methylcellulose, hydroxyethylcellulose, hypromellose phthalate, noncrystalline cellulose, magnesium aluminum silicate, triethanolamine, polyvinyl alcohol (PVA), 4-(1,1,3,3-tetramethylbutyl)-phenol polymer with ethylene oxide and formaldehyde (also known as tyloxapol, superione, and triton), poloxamers (e.g., Pluronics F68® and F108®, which are block copolymers of ethylene oxide and propylene oxide); poloxamines (e.g., Tetronic 908®, also known as Poloxamine 908®, which is a tetrafunctional block copolymer derived from sequential addition of propylene oxide and ethylene oxide to ethylenediamine (BASF Wyandotte Corporation, Parsippany, N.J.)); Tetronic 1508® (T-1508) (BASF Wyandotte Corporation), Tritons X-200®, which is an alkyl aryl polyether sulfonate (Rohm and Haas); Crodestas F-110®, which is a mixture of sucrose stearate and sucrose distearate (Croda Inc.); p-isononylphenoxypoly-(glycidol), also known as Olin-10G® or Surfactant 10-G® (Olin Chemicals, Stamford, CT); Crodestas SL-40® (Croda, Inc.); and SA90HCO, which is C18H37CH2(CON(CH3)-CH2(CHOH)4(CH20H)2 (Eastman Kodak Co.); decanoyl-N-methylglucamide; n-decyl (-D-glucopyranoside; n-decyl (-D-maltopyranoside; n-dodecyl (-D-glucopyranoside; n-dodecyl (-D-maltoside; heptanoyl-N-methylglucamide; n-heptyl-(-D-glucopyranoside; n-heptyl (-D-thioglucoside; n-hexyl (-D-glucopyranoside; nonanoyl-N-methylglucamide; n-noyl (-D-glucopyranoside; octanoyl-N-methylglucamide; n-octyl-(-D-glucopyranoside; octyl (-D-thioglucopyranoside; PEG-phospholipid, PEG-cholesterol, PEG-cholesterol derivative, PEG-vitamin A, PEG-vitamin E, lysozyme, random copolymers of vinyl pyrrolidone and vinyl acetate, and the like.

Examples of useful cationic surface stabilizers include, but are not limited to, polymers, biopolymers, polysaccharides, cellulosics, alginates, phospholipids, and nonpolymeric compounds, such as zwitterionic stabilizers, poly-n-methylpyridinium, anthryul pyridinium chloride, cationic phospholipids, chitosan, polylysine, polyvinylimidazole, polybrene, polymethylmethacrylate trimethylammoniumbromide bromide (PMMTMABr), hexyldesyltrimethylammonium bromide (HDMAB), and polyvinylpyrrolidone-2-dimethylaminoethyl methacrylate dimethyl sulfate. Other useful cationic stabilizers include, but are not limited to, cationic lipids, sulfonium, phosphonium, and quarternary ammonium compounds, such as stearyltrimethylammonium chloride, benzyl-di(2-chloroethyl)ethylammonium bromide, coconut trimethyl ammonium chloride or bromide, coconut methyl dihydroxyethyl ammonium chloride or bromide, decyl triethyl ammonium chloride, decyl dimethyl hydroxyethyl ammonium chloride or bromide, C12-15 dimethyl hydroxyethyl ammonium chloride or bromide, coconut dimethyl hydroxyethyl ammonium chloride or bromide, myristyl trimethyl ammonium methyl sulfate, lauryl dimethyl benzyl ammonium chloride or bromide, lauryl dimethyl (ethenoxy)4 ammonium chloride or bromide, N-alkyl (C12-18)dimethylbenzyl ammonium chloride, N-alkyl (C 14-18)dimethyl-benzyl ammonium chloride, N-tetradecylidmethylbenzyl ammonium chloride monohydrate, dimethyl didecyl ammonium chloride, N-alkyl and (C12-14) dimethyl 1-napthylmethyl ammonium chloride, trimethylammonium halide, alkyl-trimethylammonium salts and dialkyl-dimethylammonium salts, lauryl trimethyl ammonium chloride, ethoxylated alkyamidoalkyldialkylammonium salt and/or an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C12-14) dimethyl 1-naphthylmethyl ammonium chloride and dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C12, C15, C17 trimethyl ammonium bromides, dodecylbenzyl triethyl ammonium chloride, poly-diallyldimethylammonium chloride (DADMAC), dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride (ALIQUAT 336), POLYQUAT, tetrabutylammonium bromide, benzyl trimethylammonium bromide, choline esters (such as choline esters of fatty acids), benzalkonium chloride, stearalkonium chloride compounds (such as stearyltrimonium chloride and distearyldimonium chloride), cetyl pyridinium bromide or chloride, halide salts of quaternized polyoxyethylalkylamines, MIRAPOL and ALKAQUAT (Alkaril Chemical Company), alkyl pyridinium salts; amines, such as alkylamines, dialkylamines, alkanolamines, polyethylenepolyamines, N,N-diallcylaminoalkyl acrylates, and vinyl pyridine, amine salts, such as lauryl amine acetate, stearyl amine acetate, alkylpyridinium salt, and alkylimidazolium salt, and amine oxides; imide azolinium salts; protonated quaternary acrylamides; methylated quaternary polymers, such as poly[diallyl dimethylammonium chloride] and poly-[N-methyl vinyl pyridinium chloride]; and cationic guar.

Such exemplary cationic surface stabilizers and other useful cationic surface stabilizers are described in J. Cross and E. Singer, Cationic Surfactants: Analytical and Biological Evaluation (Marcel Dekker, 1994); P. and D. Rubingh (Editor), Cationic Surfactants: Physical Chemistry (Marcel Dekker, 1991); and J. Richmond, Cationic Surfactants: Organic Chemistry, (Marcel Dekker, 1990).

Nonpolymeric surface stabilizers are any nonpolymeric compound, such benzalkonium chloride, a carbonium compound, a phosphonium compound, an oxonium compound, a halonium compound, a cationic organometallic compound, a quarternary phosphorous compound, a pyridinium compound, an anilinium compound, an ammonium compound, a hydroxylammonium compound, a primary ammonium compound, a secondary ammonium compound, a tertiary ammonium compound, and quarternary ammonium compounds of the formula NR1R2R3R4(+). For compounds of the formula NR1R2R3R4(+):
(i) none of R1-R4 are CH3;
(ii) one of R1-R4 is CH3;
(iii) three of RI-R4 are CH3;
(iv) all of R1-R4 are CH3;
(v) two of R1-R4 are CH3, one of R1-R4 is C6H5CH2, and one of R1-R4 is an alkyl chain of seven carbon atoms or less;
(vi) two of R1-R4 are CH3, one of R1-R4 is C6H5CH2, and one of R1-R4 is an alkyl chain of nineteen carbon atoms or more;
(vii) two of R1-R4 are CH3 and one of R1-R4 is the group C6H5(CH2)n, where n>1;
(viii) two of R1-R4 are CH3, one of Rl-R4 is C6H5CH2, and one of R1-R4 comprises at least one heteroatom;
(ix) two of Rl-R4 are CH3, one of R1-R4 is C6HSCH2, and one of R1-R4 comprises at least one halogen;
(x) two of R1-R4 are CH3, one of R1-R4 is C6H5CH2, and one of R1-R4 comprises at least one cyclic fragment;
(xi) two of R1-R4 are CH3 and one of R1-R4 is a phenyl ring; or
(xii) two of R1-R4 are CH3 and two of R1-R4 are purely aliphatic fragments.

Such compounds include, but are not limited to, behenalkonium chloride, benzethonium chloride, cetylpyridinium chloride, behentrimonium chloride, lauralkonium chloride, cetalkonium chloride, cetrimonium bromide, cetrimonium chloride, cethylamine hydrofluoride, chlorallylmethenamine chloride (Quaternium-15), distearyldimonium chloride (Quaternium-5), dodecyl dimethyl ethylbenzyl ammonium chloride (Quaternium-14), Quatemium-22, Quaternium-26, Quaternium-18 hectorite, dimethylaminoethylchloride hydrochloride, cysteine hydrochloride, diethanolammonium POE (10) oletyl ether phosphate, diethanolammonium POE (3)oleyl ether phosphate, tallow alkonium chloride, dimethyl dioctadecylammoniumbentonite, stearalkonium chloride, domiphen bromide, denatonium benzoate, myristalkonium chloride, laurtrimonium chloride, ethylenediamine dihydrochloride, guanidine hydrochloride, pyridoxine HCl, iofetamine hydrochloride, meglumine hydrochloride, methylbenzethonium chloride, myrtrimonium bromide, oleyltrimonium chloride, polyquaternium-1, procainehydrochloride, cocobetaine, stearalkonium bentonite, stearalkoniumhectonite, stearyl trihydroxyethyl propylenediamine dihydrofluoride, tallowtrimonium chloride, and hexadecyltrimethyl ammonium bromide.

Most of these surface stabilizers are known pharmaceutical excipients and are described in detail in the Handbook of Pharmaceutical Excipients, published jointly by the American Pharmaceutical Association and The Pharmaceutical Society of Great Britain (The Pharmaceutical Press, 2000), specifically incorporated herein by reference.

### Povidone Polymer

Povidone polymers are preferred surface stabilizers for use in formulating an injectable nanoparticulate benzodiazepine, such as lorazepam, formulations. Povidone polymers, also known as polyvidon(e), povidonum, PVP, and polyvinylpyrrolidone, are sold under the trade names Kollidon^{®} (BASF Corp.) and Plasdone^{®} (ISP Technologies, Inc.). They are polydisperse macromolecular molecules, with a chemical name of 1-ethenyl-2-pyrrolidinone polymers and 1-vinyl-2-pyrrolidinone polymers. Povidone polymers are produced commercially as a series of products having mean molecular weights ranging from about 10,000 to about 700,000 daltons. To be useful as a surface modifier for a drug compound to be administered to a mammal, the povidone polymer must have a molecular weight of less than about 40,000 daltons, as a molecular weight of greater than 40,000 daltons would have difficulty clearing the body.

Povidone polymers are prepared by, for example, Reppe's process, comprising: (1) obtaining 1,4-butanediol from acetylene and formaldehyde by the Reppe butadiene synthesis; (2) dehydrogenating the 1,4-butanediol over copper at 200° to form γ-butyrolactone; and (3) reacting γ-butyrolactone with ammonia to yield pyrrolidone. Subsequent treatment with acetylene gives the vinyl pyrrolidone monomer. Polymerization is carried out by heating in the presence of H₂O and NH₃. *See* The Merck Index, 10th Edition, pp. 7581 (Merck & Co., Rahway, NJ, 1983).

The manufacturing process for povidone polymers produces polymers containing molecules of unequal chain length, and thus different molecular weights. The molecular weights of the molecules vary about a mean or average for each particular commercially available grade. Because it is difficult to determine the polymer's molecular weight directly, the most widely used method of classifying various molecular weight grades is by K-values, based on viscosity measurements. The K-values of various grades of povidone polymers represent a function of the average molecular weight, and are derived from viscosity measurements and calculated according to Fikentscher's formula.

The weight-average of the molecular weight, Mw, is determined by methods that measure the weights of the individual molecules, such as by light scattering. Table 1 provides molecular weight data for several commercially available povidone polymers, all of which are soluble.

**TABLE 1**

| **Povidone** | **K-Value** | **Mv (Daltons)**** | **Mw (Daltons)**** | **Mn (Daltons)**** |
|---|---|---|---|---|
| Plasdone C-15^{®} | 17±1 | 7,000 | 10,500 | 3,000 |
| Plasdone C-30^{®} | 30.5 ± 1.5 | 38,000 | 62,500* | 16,500 |
| Kollidon 12 PF^{®} | 11-14 | 3,900 | 2,000-3,000 | 1,300 |
| Kollidon 17 PF^{®} | 16-18 | 9,300 | 7,000-11,000 | 2,500 |
| Kollidon 25^{®} | 24-32 | 25,700 | 28,000-34,000 | 6,000 |

| | | | | |
|---|---|---|---|---|
| *Because the molecular weight is greater than 40,000 daltons, this povidone polymer is not useful as a surface stabilizer for a drug compound to be administered parenterally (i. e., injected). **Mv is the viscosity-average molecular weight, Mn is the number-average molecular weight, and Mw is the weight average molecular weight. Mw and Mn were determined by light scattering and ultra-centrifugation, and Mv was determined by viscosity measurements. | | | | |

Based on the data provided in Table 1, exemplary preferred commercially available povidone polymers include, but are not limited to, Plasdone C-15^{®}, Kollidon 12 PF^{®}, Kollidon 17 PF^{®}, and Kollidon 25^{®}.

### C. Nanoparticulate Benzodiazepine Particle Size

As used herein, particle size is determined on the basis of the weight average particle size as measured by conventional particle size measuring techniques well known to those skilled in the art. Such techniques include, for example, sedimentation field flow fractionation, photon correlation spectroscopy, light scattering, and disk centrifugation.

Compositions of the invention comprise benzodiazepine, such as lorazepam, nanoparticles having an effective average particle size of less than about 2000 nm *(i.e., 2* microns). In other embodiments of the invention, the benzodiazepine, such as lorazepam, nanoparticles have an effective average particle size of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 650 nm, less than about 600 nm, less than about 550 nm, less than about 500 nm, less than about 450 nm, less than about 400 nm, less than about 350 nm, less than about 300 nm, less than about 250 nm, less than about 200 nm, less than about 150 nm, less than about 100 nm, less than about 75 nm, or less than about 50 nm, as measured by light-scattering methods, microscopy, or other appropriate methods.

In another embodiment, the nanoparticulate compositions of the present invention, and the injectable nanoparticulate compositions in particular, comprise benzodiazepine, such as lorazepam, nanoparticles that have an effective average particles size of less than about 600 nm. In other embodiments, the effective average particle size is less than about 550 nm, less than about 500 nm, less than about 450 nm, less than about 400 nm, less than about 300 nm, less than about 250 nm, less than about 200 nm, less than about 150 nm, less than about 100 nm, less than about 75 nm, or less than about 50 nm.

An "effective average particle size of less than about 2000 nm" means that at least 50% of the benzodiazepine, such as lorazepam, particles have a particle size less than the effective average, by weight, i.e., less than about 2000 nm. If the "effective average particle size" is less than about 1900 nm, then at least about 50% of the benzodiazepine, such as lorazepam, particles have a size of less than about 1900 nm, when measured by the above-noted techniques. The same is true for the other particle sizes referenced above. In other embodiments, at least about 70%, at least about 90%, at least about 95%, or at least about 99% of the benzodiazepine, such as lorazepam, particles have a particle size less than the effective average, *i.e*., less than about 2000 nm, about 1900 nm, about 1800 nm, *etc..*

In the present invention, the value for D50 of a nanoparticulate benzodiazepine, such as lorazepam, composition is the particle size below which 50% of the benzodiazepine, such as lorazepam, particles fall, by weight. Similarly, D90 is the particle size below which 90% of the benzodiazepine, such as lorazepam, particles fall, by weight.

### D. Concentration of Nanoparticulate Benzodiazepine and Surface Stabilizers

The relative amounts of benzodiazepine, such as lorazepam, and one or more surface stabilizers can vary widely. The optimal amount of the individual components depends, for example, upon physical and chemical attributes of the surface stabilizer(s) and benzodiazepine selected, such as the hydrophilic lipophilic balance (HLB), melting point, and the surface tension of water solutions of the stabilizer and benzodiazepine, etc.

Preferably, the concentration of benzodiazepine, such as lorazepam, can vary from about 99.5% to about 0.001 %, from about 95% to about 0.1 %, or from about 90% to about 0.5%, by weight, based on the total combined weight of the benzodiazepine and at least one surface stabilizer, not including other excipients. Higher concentrations of the active ingredient are generally preferred from a dose and cost efficiency standpoint.

Preferably, the concentration of surface stabilizer can vary from about 0.5% to about 99.999%, from about 5.0% to about 99.9%, or from about 10% to about 99.5%, by weight, based on the total combined dry weight of benzodiazepine, such as lorazepam, and at least one surface stabilizer, not including other excipients.

### E. Other Pharmaceutical Excipients

Pharmaceutical compositions of the invention may also comprise one or more binding agents, filling agents, lubricating agents, suspending agents, sweeteners, flavoring agents, preservatives, buffers, wetting agents, disintegrants, effervescent agents, and other excipients depending upon the route of administration and the dosage form desired. Such excipients are well known in the art.

Examples of filling agents are lactose monohydrate, lactose anhydrous, and various starches; examples of binding agents are various celluloses and cross-linked polyvinylpyrrolidone, microcrystalline cellulose, such as Avicel^{®} PH101 and Avicel^{®} PH102, microcrystalline cellulose, and silicified microcrystalline cellulose (ProSolv SMCC™).

Suitable lubricants, including agents that act on the flowability of the powder to be compressed, are colloidal silicon dioxide, such as Aerosil^{®} 200, talc, stearic acid, magnesium stearate, calcium stearate, and silica gel.

Examples of sweeteners are any natural or artificial sweetener, such as sucrose, xylitol, sodium saccharin, cyclamate, aspartame, and acsulfame. Examples of flavoring agents are Magnasweet^{®} (trademark of MAFCO), bubble gum flavor, and fruit flavors, and the like.

Examples of preservatives are potassium sorbate, methylparaben, propylparaben, benzoic acid and its salts, other esters of parahydroxybenzoic acid such as butylparaben, alcohols such as ethyl or benzyl alcohol, phenolic compounds such as phenol, and quarternary compounds such as benzalkonium chloride.

Suitable diluents include pharmaceutically acceptable inert fillers, such as microcrystalline cellulose, lactose, dibasic calcium phosphate, saccharides, and/or mixtures of any of the foregoing. Examples of diluents include microcrystalline cellulose, such as Avicel^{®} PH101 and Avicel^{®} PH102; lactose such as lactose monohydrate, lactose anhydrous, and Pharmatose^{®} DCL21; dibasic calcium phosphate such as Emcompress^{®}; mannitol; starch; sorbitol; sucrose; and glucose.

Suitable disintegrants include lightly crosslinked polyvinyl pyrrolidone, corn starch, potato starch, maize starch, and modified starches, croscarmellose sodium, cross-povidone, sodium starch glycolate, and mixtures thereof.

Examples of effervescent agents are effervescent couples, such as an organic acid and a carbonate or bicarbonate. Suitable organic acids include, for example, citric, tartaric, malic, fumaric, adipic, succinic, and alginic acids and anhydrides and acid salts. Suitable carbonates and bicarbonates include, for example, sodium carbonate, sodium bicarbonate, potassium carbonate, potassium bicarbonate, magnesium carbonate, sodium glycine carbonate, L-lysine carbonate, and arginine carbonate. Alternatively, only the sodium bicarbonate component of the effervescent couple may be present.

### F. Aerosol Formulations of Nanoparticulate Benzodiazepines

The compositions of the invention encompass aerosols comprising a nanoparticulate benzodiazepine, such as lorazepam. Aerosols can be defined as colloidal systems comprising very finely divided liquid droplets or dry particles dispersed in and surrounded by a gas. Both liquid and dry powder aerosol compositions are encompassed by the invention.

Aerosols intended for delivery to the nasal mucosa are inhaled through the nose. For optimal delivery to the nasal cavities, droplet or aggregate dry powder particle sizes of about 5 to about 100 microns are useful, with droplet or aggregate dry powder particle sizes of about 30 to about 60 microns being preferred. The nanoparticulate benzodiazepine particles are either suspended in the liquid droplet for an aqueous dispersion aerosol, or comprised in the aggregate dry powder particles for a dry powder aerosol. For nasal delivery, a larger inhaled particle size is desired to maximize impaction on the nasal mucosa and to minimize or prevent pulmonary deposition of the administered formulation. Inhaled particles may be defined as (1) liquid droplets comprising a suspended benzodiazepine particle, such as lorazepam, (2) dry particles of a benzodiazepine, such as lorazepam, (3) dry powder aggregates of a nanoparticulate benzodiazepine, such as lorazepam, or (4) dry particles of a diluent which comprise an embedded benzodiazepine, such as lorazepam, nanoparticles.

For delivery to the upper respiratory region, inhaled particle sizes of about 2 to about 10 microns are preferred. More preferred is about 2 to about 6 microns. Delivery to the upper respiratory region may be desirable for a nanoparticulate benzodiazepine, such as lorazepam nanoparticles, that are to act locally. This is because a nanoparticulate benzodiazepine, such as lorazepam, deposited in the upper respiratory tract can dissolve and act on the smooth muscle of the airway, rather than being absorbed into the bloodstream of the patient. However, the goal for an inhaled benzodiazepine, such as lorazepam, is systemic delivery, such as in cases of a benzodiazepine, such as lorazepam, which are not amenable to oral administration. It is preferred that a benzodiazepine, such as lorazepam, which is intended for systemic administration, be delivered to the alveolar region of the lung because 99.99% of the available surface area for a benzodiazepine, such as lorazepam, absorption is located in the peripheral alveoli. Thus, with administration to the alveolar region, rapid absorption can be realized. For delivery to the deep lung (alveolar) region, inhaled particle sizes of less than about 2 microns are preferred.

### 1. Concentration of Nanoparticulate Benzodiazepine

For aqueous aerosol formulations, nanoparticulate benzodiazepine, such as lorazepam, nanoparticles are present at a concentration of about 0.05 mg/mL up to about 600 mg/mL. For dry powder aerosol formulations, nanoparticulate benzodiazepine, such as lorazepam, nanoparticles are present at a concentration of about 0.05 mg/g up to about 990 mg/g, depending on the desired dosage. Concentrated nanoparticulate aerosols, defined as comprising a nanoparticulate benzodiazepine, such as lorazepam, at a concentration of about 10 mg/mL up to about 600 mg/mL for aqueous aerosol formulations, and about 10 mg/g up to about 990 mg/g for dry powder aerosol formulations, are specifically encompassed by the present invention. More concentrated aerosol formulations enable the delivery of large quantities of a nanoparticulate benzodiazepine, such as nanoparticulate lorazepam, to the lung in a very short period of time, thereby providing effective delivery to appropriate areas of the lung or nasal cavities in short administration times, i.e., less than about 15 seconds as compared to administration times of up to 4 to 20 minutes as found in conventional pulmonary nebulizer therapies.

### 2. Aqueous Aerosols

The present invention encompasses aqueous formulations comprising nanoparticulate benzodiazepine, such as lorazepam, nanoparticles. Aqueous formulations of the invention comprise colloidal dispersions of a poorly water-soluble nanoparticulate benzodiazepine, such as lorazepam, in an aqueous vehicle which are aerosolized using air-jet or ultrasonic nebulizers. The advantages of the invention can best be understood by comparing the sizes of nanoparticulate and conventional micronized benzodiazepine, such as lorazepam, particles with the sizes of liquid droplets produced by conventional nebulizers. Conventional micronized material is generally about 2 to about 5 microns or more in diameter and is approximately the same size as the liquid droplet size produced by medical nebulizers. In contrast, nanoparticulate benzodiazepine, such as lorazepam, are substantially smaller than the droplets in such an aerosol. Thus, aerosols comprising nanoparticulate benzodiazepine, such as lorazepam, improve drug delivery efficiency. Such aerosols comprise a higher number of nanoparticles per unit dose, resulting in each aerosolized droplet containing active benzodiazepine, such as lorazepam.

Thus, with administration of the same dosages of nanoparticulate and micronized benzodiazepine, such as lorazepam, more lung or nasal cavity surface area is covered by the aerosol formulation comprising a nanoparticulate benzodiazepine, such as lorazepam.

Another advantage of the invention is that the compositions of the invention permit a poorly water-soluble benzodiazepine, such as lorazepam, to be delivered to the deep lung. Conventional micronized drug substance is too large to reach the peripheral lung regardless of the size of the droplet produced by the nebulizer, but the present invention permits nebulizers which generate very small (about 0.5 to about 2 microns) aqueous droplets to deliver a poorly water-soluble benzodiazepine, such as lorazepam, in the form of nanoparticles to the alveoli. One example of such devices is the Circular™ aerosol (Westmed Corp., Tucson, Ariz.).

Yet another advantage of the invention is that ultrasonic nebulizers can be used to deliver a poorly water-soluble benzodiazepine, such as lorazepam, to the lung. Unlike conventional micronized material, nanoparticulate benzodiazepine, such as lorazepam, are readily aerosolized and show *good in vitro* deposition characteristics. A specific advantage of the invention is that it permits poorly water-soluble benzodiazepine, such as lorazepam, to be aerosolized by ultrasonic nebulizers which require a nanoparticulate benzodiazepine, such as lorazepam, to pass through very fine orifices to control the size of the aerosolized droplets. While conventional drug material would be expected to occlude the pores, such nanoparticulates are much smaller and can pass through the pores without difficulty.

Another advantage of the invention is the enhanced rate of dissolution of a poorly water-soluble benzodiazepine, such as lorazepam, which is practically insoluble in water. Since dissolution rate is a function of the total surface area of a benzodiazepine, such as lorazepam, to be dissolved, a more finely divided benzodiazepine (e.g., nanoparticles) have much faster dissolution rates than conventional micronized drug particles. This can result in more rapid absorption of an inhaled benzodiazepine, such as lorazepam. For a nasally administered benzodiazepine, such as lorazepam, it can result in more complete absorption of the dose, since with a nanoparticulate dose of the benzodiazepine, such as lorazepam, the nanoparticles can dissolve rapidly and completely before being cleared by the mucociliary mechanism.

### 3. Dry Powder Aerosol Formulations

Another embodiment of the invention is directed to dry powder aerosol formulations comprising a benzodiazepine, such as lorazepam, for pulmonary and/or nasal administration. Dry powders, which can be used in both DPIs and pMDIs, can be made by spray-drying an aqueous nanoparticulate dispersion of a benzodiazepine, such as lorazepam. Alternatively, dry powders comprising a nanoparticulate benzodiazepine, such as lorazepam, can be made by freeze-drying dispersions of the nanoparticles. Combinations of the spray-dried and freeze-dried nanoparticulate powders can be used in DPIs and pMDIs. For dry powder aerosol formulations, a nanoparticulate benzodiazepine, such as lorazepam, may be present at a concentration of about 0.05 mg/g up to about 990 mg/g. In addition, the more concentrated aerosol formulations *(i.e.,* for dry powder aerosol formulations about 10 mg/g up to about 990 mg/g) have the additional advantage of enabling large quantities of a benzodiazepine, such as lorazepam, to be delivered to the lung in a very short period of time, e.g., about 1 to about 2 seconds (1 puff).

The invention is also directed to dry powders which comprise nanoparticulate compositions for pulmonary or nasal delivery. The powders may comprise inhalable aggregates of a nanoparticulate benzodiazepine, such as lorazepam, or inhalable particles of a diluent which comprises at least one embedded benzodiazepine, such as lorazepam. Powders comprising a nanoparticulate benzodiazepine, such as lorazepam, can be prepared from aqueous dispersions of nanoparticles by removing the water by spray-drying or lyophilization (freeze drying). Spray-drying is less time consuming and less expensive than freeze-drying, and therefore more cost-effective. However, certain benzodiazepines, such as lorazepam, benefit from lyophilization rather than spray-drying in making dry powder formulations.

Dry powder aerosol delivery devices must be able to accurately, precisely, and repeatably deliver the intended amount of benzodiazepine, such as lorazepam. Moreover, such devices must be able to fully disperse the dry powder into individual particles of a respirable size. Conventional micronized drug particles of 2-3 microns in diameter are often difficult to meter and disperse in small quantities because of the electrostatic cohesive forces inherent in such powders. These difficulties can lead to loss of drug substance to the delivery device as well as incomplete powder dispersion and sub-optimal delivery to the lung. Many drug compounds, particularly a benzodiazepine, such as lorazepam, are intended for deep lung delivery and systemic absorption. Since the average particle sizes of conventionally prepared dry powders are usually in the range of 2-3 microns, the fraction of material which actually reaches the alveolar region may be quite small. Thus, delivery of micronized dry powders to the lung, especially the alveolar region, is generally very inefficient because of the properties of the powders themselves.

The dry powder aerosols which comprise nanoparticulate benzodiazepine, such as lorazepam, can be made smaller than comparable micronized drug substance and, therefore, are appropriate for efficient delivery to the deep lung. Moreover, aggregates of nanoparticulate benzodiazepine, such as lorazepam, are spherical in geometry and have good flow properties, thereby aiding in dose metering and deposition of the administered composition in the lung or nasal cavities.

Dry nanoparticulate compositions can be used in both DPIs and pMDIs. (In this invention, "dry" refers to a composition having less than about 5% water.)

### a. Spray-dried powders comprising a nanoparticulate benzodiazepine

Powders comprising a nanoparticulate benzodiazepine, such as lorazepam, can be made by spray-drying aqueous dispersions of a nanoparticulate benzodiazepine, such as lorazepam, and a surface stabilizer to form a dry powder which comprises aggregated nanoparticulate benzodiazpine, such as lorazepam. The aggregates can have a size of about 1 to about 2 microns which is suitable for deep lung delivery. The aggregate particle size can be increased to target alternative delivery sites, such as the upper bronchial region or nasal mucosa by increasing the concentration of a benzodiazepine, such as lorazepam, in the spray-dried dispersion or by increasing the droplet size generated by the spray dryer.

Alternatively, the aqueous dispersion of a nanoparticulate benzodiazepine, such as lorazepam, and surface stabilizer can comprise a dissolved diluent such as lactose or mannitol which, when spray dried, forms inhalable diluent particles, each of which comprises at least one embedded benzodiazepine, such as lorazepam, nanoparticle and surface stabilizer. The diluent particles with an embedded benzodiazepine, such as lorazepam, nanoparticles can have a particle size of about 1 to about 2 microns, suitable for deep lung delivery. In addition, the diluent particle size can be increased to target alternate delivery sites, such as the upper bronchial region or nasal mucosa by increasing the concentration of dissolved diluent in the aqueous dispersion prior to spray drying, or by increasing the droplet size generated by the spray dryer.

Spray-dried powders can be used in DPIs or pMDIs, either alone or combined with freeze-dried nanoparticulate active agent powder. In addition, spray-dried powders comprising a nanoparticulate benzodiazepine, such as lorazepam, can be reconstituted and used in either jet or ultrasonic nebulizers to generate aqueous dispersions having respirable droplet sizes, where each droplet comprises at least one nanoparticulate benzodiazepine, such as lorazepam. Concentrated nanoparticulate dispersions may also be used in these aspects of the invention.

### b. Freeze-Dried Powders Comprising a Nanoparticulate Benzodiazepine

Nanoparticulate benzodiazepine, such as lorazepam, dispersions can also be freeze-dried to obtain powders suitable for nasal or pulmonary delivery. Such powders may comprise aggregated nanoparticulate benzodiazepine, such as lorazepam, having a surface stabilizer. Such aggregates may have sizes within a respirable range, *i.e.,* about 2 to about 5 microns. Larger aggregate particle sizes can be obtained for targeting alternate delivery sites, such as the nasal mucosa.

Freeze dried powders of the appropriate particle size can also be obtained by freeze drying aqueous dispersions of benzodiazepine, such as lorazepam, and surface stabilizer, which additionally may comprise a dissolved diluent such as lactose or mannitol. In these instances the freeze dried powders comprise respirable particles of diluent, each of which comprises at least one embedded nanoparticulate benzodiazepine, such as lorazepam.

Freeze-dried powders can be used in DPIs or pMIs, either alone or combined with spray-dried nanoparticulate powder. In addition, freeze-dried powders containing a nanoparticulate benzodiazepine, such as lorazepam, can be reconstituted and used in either jet or ultrasonic nebulizers to generate aqueous dispersions having respirable droplet sizes, where each droplet comprises at least one nanoparticulate benzodiazepine, such as lorazepam. Concentrated nanoparticulate dispersions may also be used in these aspects of the invention.

### c. Propellant-Based Aerosols

Yet another embodiment of the invention is directed to a process and composition for propellant-based systems comprising a nanoparticulate benzodiazepine, such as lorazepam. Such formulations may be prepared by wet milling the coarse benzodiazepine, and preferably, lorazepam particles and surface stabilizer in liquid propellant, either at ambient pressure or under high pressure conditions. Alternatively, dry powders comprising a nanoparticulate benzodiazepine, such as lorazepam, may be prepared by spray-drying or freeze-drying aqueous dispersions of a nanoparticulate benzodiazepine, such as lorazepam, with the resultant powders dispersed into suitable propellants for use in conventional pMDIs. Such nanoparticulate pMDI formulations can be used for either nasal or pulmonary delivery. For pulmonary administration, such formulations afford increased delivery to the deep lung regions because of the small *(i.e.,* about 1 to about 2 microns) particle sizes available from these methods. Concentrated aerosol formulations can also be employed in pMDIs.

Another embodiment of the invention is directed to a process and composition for propellant-based MDIs containing nanoparticulate benzodiazepine, such as lorazepam. pMDIs can comprise either the discrete nanoparticles and surface stabilizer, aggregates of the nanoparticles and surface stabilizer, or diluent particles comprising the embedded nanoparticles. pMDIs can be used for targeting the nasal cavity, the conducting airways of the lung, or the alveoli. Compared to conventional formulations, the present invention affords increased delivery to the deep lung regions because the inhaled nanoparticles are smaller than conventional micronized material (<2 microns) and are distributed over a larger mucosal or alveolar surface area as compared to miconized drugs.

The nanoparticulate drug pMDIs of the invention can utilize either chlorinated or non-chlorinated propellants. Concentrated nanoparticulate aerosol formulations can also be employed in pMDIs.

In a non-aqueous, non-pressurized milling system, a non-aqueous liquid which has a vapor pressure of 1 atm or less at room temperature is used as a milling medium and may be evaporated to yield a dry nanoparticulate benzodiazepine, and preferably, lorazepam nanoparticles and surface modifier. The non-aqueous liquid may be, for example, a high-boiling halogenated hydrocarbon. The dry nanoparticulate benzodiazepine, and preferably, lorazepam nanoparticle composition thus produced may then be mixed with a suitable propellant or propellants and used in a conventional pMDI.

Alternatively, in a pressurized milling operation, a non-aqueous liquid which has a vapor pressure >1 atm at room temperature is used as a milling medium for making a nanoparticulate benzodiazepine, such as lorazepam, and surface stabilizer composition. Such a liquid may be, for example, a halogenated hydrocarbon propellant which has a low boiling point. The resultant nanoparticulate composition can then be used in a conventional pMDI without further modification, or can be blended with other suitable propellants. Concentrated aerosols may also be made by such methods.

### G. Injectable Nanoparticulate Benzodiazepine Formulations

The invention provides injectable nanoparticulate benzodiazepine, such as lorazepam, formulations that can comprise high drug concentrations in low injection volumes, with rapid drug dissolution upon administration. In addition, the injectable nanoparticulate benzodiazepine, such as lorazepam, formulations of the invention eliminate the need to use polyoxyl 60 hydrogenated castor oil (HCO-60) as a solubilizer. An exemplary injectable composition comprises, based on % w/w:

| | |
|---|---|
| benzodiazepine (such as lorazepam) | 5 - 50% |
| povidone polymer | 0.1- 50% |
| preservatives | 0.05 - 0.25% |
| pH adjusting agent | pH about 6 to about 7 |
| water for injection | q.s. |

Exemplary preservatives include methylparaben (about 0.18% based on % w/w), propylparaben (about 0.02% based on % w/w), phenol (about 0.5% based on % w/w), and benzyl alcohol (up to 2% v/v). An exemplary pH adjusting agent is sodium hydroxide, and an exemplary liquid carrier is sterile water for injection. Other useful preservatives, pH adjusting agents, and liquid carriers are well-known in the art.

### III. Methods Of Making the Benzodiazepine Formulations

Nanoparticulate benzodiazepine, such as lorazepam, compositions can be made using any suitable method known in the art such as, for example, milling, homogenization, precipitation, or supercritical fluid techniques. Exemplary methods of making nanoparticulate compositions are described in U.S. Patent No. 5,145,684. Methods of making nanoparticulate compositions are also described in U.S. Patent No. 5,518,187 for "Method of Grinding Pharmaceutical Substances;" U.S. Patent No. 5,718,388 for "Continuous Method of Grinding Pharmaceutical Substances;" U.S. Patent No. 5,862,999 for "Method of Grinding Pharmaceutical Substances;" U.S. Patent No. 5,665,331 for "Co-Microprecipitation ofNanoparticulate Pharmaceutical Agents with Crystal Growth Modifiers;" U.S. Patent No. 5,662,883 for "Co-Microprecipitation ofNanoparticulate Pharmaceutical Agents with Crystal Growth Modifiers;" U.S. Patent No. 5,560,932 for "Microprecipitation ofNanoparticulate Pharmaceutical Agents;" U.S. Patent No. 5,543,133 for "Process of Preparing X-Ray Contrast Compositions Containing Nanoparticles;" U.S. Patent No. 5,534,270 for "Method of Preparing Stable Drug Nanoparticles;" U.S. Patent No. 5,510,118 for "Process of Preparing Therapeutic Compositions Containing Nanoparticles;" and U.S. Patent No. 5,470,583 for "Method of Preparing Nanoparticle Compositions Containing Charged Phospholipids to Reduce Aggregation," all of which are specifically incorporated herein by reference.

The resultant nanoparticulate benzodiazepine, such as lorazepam, compositions or dispersions can be utilized in injectable, aerosol dosage formulations, controlled release formulations, lyophilized formulations, delayed release formulations, extended release formulations, pulsatile release formulations, mixed immediate release and controlled release formulations, etc.

Consistent with the above disclosure, provided herein is a method of preparing the nanoparticulate benzodiazepine, such as lorazepam, formulations of the invention. The method comprises the steps of: (1) dispersing a benzodiazepine, such as lorazepam, in a liquid dispersion media; and (2) mechanically reducing the particle size of the benzodiazepine, such as lorazepam, to the desired effective average particle size, such as less than about 2000 nm or less than about 600 nm. A surface stabilizer can be added before, during, or after particle size reduction of the benzodiazepine, such as lorazepam. The liquid dispersion media can be maintained at a physiologic pH, for example, within the range of from about 3.0 to about 8.0 during the size reduction process; more preferably within the range of from about 5.0 to about 7.5 during the size reduction process. The dispersion media used for the size reduction process is preferably aqueous, although any media in which the benzodiazepine, such as lorazepam, is poorly soluble and dispersible can be used, such as safflower oil, ethanol, t-butanol, glycerin, polyethylene glycol (PEG), hexane, or glycol.

Effective methods of providing mechanical force for particle size reduction of a benzodiazepine, such as lorazepam, include ball milling, media milling, and homogenization, for example, with a Microfluidizer^{®} (Microfluidics Corp.). Ball milling is a low energy milling process that uses milling media, drug, stabilizer, and liquid. The materials are placed in a milling vessel that is rotated at optimal speed such that the media cascades and reduces the drug particle size by impaction. The media used must have a high density as the energy for the particle reduction is provided by gravity and the mass of the attrition media.

Media milling is a high energy milling process. Drug, stabilizer, and liquid are placed in a reservoir and recirculated in a chamber containing media and a rotating shaft/impeller. The rotating shaft agitates the media which subjects the drug to impaction and sheer forces, thereby reducing the drug particle size.

Homogenization is a technique that does not use milling media. Drug, stabilizer, and liquid (or drug and liquid with the stabilizer added after particle size reduction) constitute a process stream propelled into a process zone, which in the Mcrofluidizer^{®} is called the Interaction Chamber. The product to be treated is inducted into the pump, and then forced out. The priming valve of the Microfluidizer^{®} purges air out of the pump. Once the pump is filled with product, the priming valve is closed and the product is forced through the interaction chamber. The geometry of the interaction chamber produces powerful forces of sheer, impact, and cavitation which are responsible for particle size reduction. Specifically, inside the interaction chamber, the pressurized product is split into two streams and accelerated to extremely high velocities. The formed jets are then directed toward each other and collide in the interaction zone. The resulting product has very fine and uniform particle or droplet size. The Microfluidizer^{®} also provides a heat exchanger to allow cooling of the product. U.S. Patent No. 5,510,118, which is specifically incorporated by reference, refers to a process using a Microfluidizer.^{®}

Using a particle size reduction method, the particle size of benzodiazepine, such as lorazepam, is reduced to the desired effective average particle size, such as less than about 2000 nm for the aerosol formulation, and less than about 600 nm for the injectable formulation.

The benzodiazepine, such as lorazepam, can be added to a liquid media in which it is essentially insoluble to form a premix. The concentration of the benzodiazepine, such as lorazepam, in the liquid media can vary from about 5 to about 60%, and preferably is from about 15 to about 50% (w/v), and more preferably about 20 to about 40%. The surface stabilizer can be present in the premix or it can be added to the drug dispersion following particle size reduction. The concentration of the surface stabilizer can vary from about 0.1 to about 50%, and preferably is from about 0.5 to about 20%, and more preferably from about 1 to about 10%, by weight.

The premix can be used directly by subjecting it to mechanical means to reduce the average benzodiazepine, such as lorazepam, particle size in the dispersion to less than about 2000 nm. It is preferred that the premix be used directly when a ball mill is used for attrition. Alternatively, the benzodiazepine, such as lorazepam, and at least one surface stabilizer can be dispersed in the liquid media using suitable agitation, e.g., a Cowles type mixer, until a homogeneous dispersion is observed in which there are no large agglomerates visible to the naked eye. It is preferred that the premix be subjected to such a premilling dispersion step when a recirculating media mill is used for attrition.

The mechanical means applied to reduce the benzodiazepine, such as lorazepam, particle size conveniently can take the form of a dispersion mill. Suitable dispersion mills include a ball mill, an attritor mill, a vibratory mill, and media mills such as a sand mill and a bead mill. A media mill is preferred due to the relatively shorter milling time required to provide the desired reduction in particle size. For media milling, the apparent viscosity of the premix is preferably from about 100 to about 1000 centipoise, and for ball milling the apparent viscosity of the premix is preferably from about 1 up to about 100 centipoise. Such ranges tend to afford an optimal balance between efficient particle size reduction and media erosion.

The attrition time can vary widely and depends primarily upon the particular mechanical means and processing conditions selected. For ball mills, processing times of up to five days or longer may be required. Alternatively, processing times of less than 1 day (residence times of one minute up to several hours) are possible with the use of a high shear media mill.

The benzodiazepine, such as lorazepam, particles can be reduced in size at a temperature which does not significantly degrade the benzodiazepine, such as lorazepam. Processing temperatures of less than about 30 to less than about 40°C are ordinarily preferred. If desired, the processing equipment can be cooled with conventional cooling equipment. Control of the temperature, e.g., by jacketing or immersion of the milling chamber in ice water, is contemplated. Generally, the method of the invention is conveniently carried out under conditions of ambient temperature and at processing pressures which are safe and effective for the milling process. Ambient processing pressures are typical of ball mills, attritor mills, and vibratory mills.

### Grinding Media

The grinding media can comprise particles that are preferably substantially spherical in shape, e.g., beads, consisting essentially of polymeric resin. Alternatively, the grinding media can comprise a core having a coating of a polymeric resin adhered thereon. The polymeric resin can have a density from about 0.8 to about 3.0 g/cm³.

In general, suitable polymeric resins are chemically and physically inert, substantially free of metals, solvent, and monomers, and of sufficient hardness and friability to enable them to avoid being chipped or crushed during grinding. Suitable polymeric resins include crosslinked polystyrenes, such as polystyrene crosslinked with divinylbenzene; styrene copolymers; polycarbonates; polyacetals, such as Delrin^{®} (E.I. du Pont de Nemours and Co.); vinyl chloride polymers and copolymers; polyurethanes; polyamides; poly(tetrafluoroethylenes), e.g., Teflon^{®}(E.I. du Pont de Nemours and Co.), and other fluoropolymers; high density polyethylenes; polypropylenes; cellulose ethers and esters such as cellulose acetate; polyhydroxymethacrylate; polyhydroxyethyl acrylate; and silicone-containing polymers such as polysiloxanes and the like. The polymer can be biodegradable. Exemplary biodegradable polymers include poly(lactides), poly(glycolide) copolymers of lactides and glycolide, polyanhydrides, poly(hydroxyethyl methacylate), poly(imino carbonates), poly(N-acylhydroxyproline)esters, poly(N-palmitoyl hydroxyproline) esters, ethylene-vinyl acetate copolymers, poly(orthoesters), poly(caprolactones), and poly(phosphazenes). For biodegradable polymers, contamination from the media itself advantageously can metabolize *in vivo* into biologically acceptable products that can be eliminated from the body.

The grinding media preferably ranges in size from about 0.01 to about 3 mm. For fine grinding, the grinding media is preferably from about 0.02 to about 2 mm, and more preferably from about 0.03 to about 1 mm in size.

In a preferred grinding process the particles are made continuously. Such a method comprises continuously introducing a benzodiazepine, such as lorazepam, into a milling chamber, contacting the benzodiazepine, such as lorazepam, with grinding media while in the chamber to reduce the benzodiazepine particle size, and continuously removing the nanoparticulate benzodiazepine from the milling chamber.

The grinding media is separated from the milled nanoparticulate benzodiazepine, such as lorazepam, using conventional separation techniques, in a secondary process such as by simple filtration, sieving through a mesh filter or screen, and the like. Other separation techniques such as centrifugation may also be employed.

### Sterile Product Manufacturing

Development of injectable compositions requires the production of a sterile product. The manufacturing process of the present invention is similar to typical known manufacturing processes for sterile suspensions. A typical sterile suspension manufacturing process flowchart is as follows:

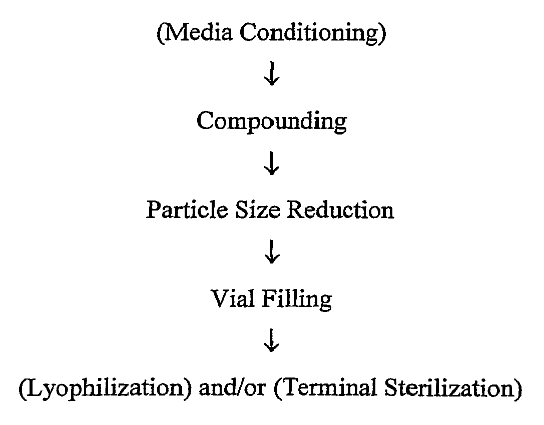

As indicated by the optional steps in parentheses, some of the processing is dependent upon the method of particle size reduction and/or method of sterilization. For example, media conditioning is not required for a milling method that does not use media. If terminal sterilization is not feasible due to chemical and/or physical instability, aseptic processing can be used.

### Aet-osol Formulations

A nanoparticulate benzodiazepine, such as lorazepam, composition for aerosol administration can be made by, for example, by (1) nebulizing an aqueous dispersion of nanoparticulate benzodiazepine, such as lorazepam, obtained by milling, homogenization, precipitation, or supercritical fluid processes; (2) aerosolizing a dry powder of aggregates of nanoparticulate benzodiazepine, such as lorazepam, and surface modifier (the aerosolized composition may additionally contain a diluent); or (3) aerosolizing a suspension of a nanoparticulate benzodiazepine, such as lorazepam, aggregates in a non-aqueous propellant. The aggregates of nanoparticulate benzodiazepine, such as lorazepam, and surface stabilizer, which may additionally contain a diluent, can be made in a non-pressurized or a pressurized non-aqueous system. Concentrated aerosol formulations may also be made by such methods.

### A. Aqueous Milling to Obtain Nanoparticulate Benzodiazepine Dispersions

In an exemplary aqueous milling process, benzodiazepine, such as lorazepam, particles are dispersed in a liquid dispersion media and mechanical means is applied in the presence of grinding media to reduce the particle size of the benzodiazepine, such as lorazepam, to the desired effective average particle size. The particles can be reduced in size in the presence of one or more surface stabilizers. Alternatively, the particles can be contacted with one or more surface stabilizer either before or after attrition. Other compounds, such as a diluent, can be added to the benzodiazepine, such as lorazepam, and surface stabilizer composition during the size reduction process. Dispersions can be manufactured continuously or in a batch mode.

### B. Precipitation to Obtain Nanoparticulate Benzodiazepine Compositions

Another method of forming the desired nanoparticle dispersion is by microprecipitation. This is a method of preparing stable dispersions of nanoparticulate benzodiazepine, such as lorazepam, in the presence of one or more surface stabilizers and one or more colloid stability enhancing surface active agents free of any trace toxic solvents or solubilized heavy metal impurities. Such a method comprises, for example, (1) dissolving the benzodiazepine, such as lorazepam, in a suitable solvent with mixing; (2) adding the formulation from step (1) with mixing to a solution comprising at least one surface stabilizer to form a clear solution; and (3) precipitating the formulation from step (2) with mixing using an appropriate nonsolvent. The method can be followed by removal of any formed salt, if present, by dialysis or diafiltration and concentration of the dispersion by conventional means. The resultant nanoparticulate benzodiazepine, such as lorazepam, dispersion can be utilized in liquid nebulizers or processed to form a dry powder for use in a DPI or pMDI.

### C. Non-Aqueous Non-Pressurized Milling System

In a non-aqueous, non-pressurized milling system, a non-aqueous liquid having a vapor pressure of about 1 atm or less at room temperature and in which the benzodiazepine, such as lorazepam, is essentially insoluble is used as a wet milling media to make a nanoparticulate benzodiazepine, such as lorazepam, composition. In such a process, a slurry of benzodiazepine, such as lorazepam, and surface stabilizer is milled in the non-aqueous media to generate nanoparticulate benzodiazepine, such as lorazepam. Examples of suitable non-aqueous media include ethanol, trichloromonofluoromethane, (CFC-11), and dichlorotetrafluoroethane (CFC-114). An advantage of using CFC-11 is that it can be handled at only marginally cool room temperatures, whereas CFC-114 requires more controlled conditions to avoid evaporation. Upon completion of milling the liquid medium may be removed and recovered under vacuum or heating, resulting in a dry nanoparticulate benzodiazepine, and preferably, lorazepam nanoparticle composition. The dry composition may then be filled into a suitable container and charged with a final propellant. Exemplary final product propellants, which ideally do not contain chlorinated hydrocarbons, include HFA-134a (tetrafluoroethane) and HFA-227 (heptafluoropropane). While non-chlorinated propellants may be preferred for environmental reasons, chlorinated propellants may also be used in this aspect of the invention.

### D. Non-Aqueous Pressurized Milling System

In a non-aqueous, pressurized milling system, a non-aqueous liquid media having a vapor pressure significantly greater than 1 atm at room temperature is used in the milling process to make nanoparticulate benzodiazepine, such as lorazepam, compositions. If the milling media is a suitable halogenated hydrocarbon propellant, the resultant dispersion may be filled directly into a suitable pMDI container. Alternately, the milling media can be removed and recovered under vacuum or heating to yield a dry benzodiazepine, such as lorazepam, nanoparticulate composition. This composition can then be filled into an appropriate container and charged with a suitable propellant for use in a pMDI.

### E. Spray-Dried Powder Aerosol Formulations

Spray drying is a process used to obtain a powder comprising nanoparticulate drug particles following particle size reduction of the benzodiazepine, such as lorazepam, in a liquid media. In general, spray-drying is used when the liquid media has a vapor pressure of less than about 1 atm at room temperature. A spray-dryer is a device which allows for liquid evaporation and powder collection. A liquid sample, either a solution or suspension, is fed into a spray nozzle. The nozzle generates droplets of the sample within a range of about 20 to about 100 µm in diameter which are then transported by a carrier gas into a drying chamber. The carrier gas temperature is typically between about 80 and about 200 degrees C. The droplets are subjected to rapid liquid evaporation, leaving behind dry particles which are collected in a special reservoir beneath a cyclone apparatus.

If the liquid sample comprises an aqueous dispersion of a nanoparticulate benzodiazepine, such as lorazepam, and surface stabilizer, the collected product will comprise spherical aggregates of the nanoparticulate benzodiazepine, such as lorazepam. If the liquid sample comprises an aqueous dispersion of nanoparticles in which an inert diluent material was dissolved (such as lactose or mannitol), the collected product will comprise diluent (e.g., lactose or mannitol) particles which comprise embedded nanoparticulate benzodiazepine, such as lorazepam. The final size of the collected product can be controlled and depends on the concentration of nanoparticulate benzodiazepine, such as lorazepam, and/or diluent in the liquid sample, as well as the droplet size produced by the spray-dryer nozzle. For deep lung delivery it is desirable for the collected product size to be less than about 2 microns in diameter, for delivery to the conducting airways it is desirable for the collected product size to be about 2 to about 6 microns in diameter, and for nasal delivery a collected product size of about 5 to about 100 microns is preferred. Collected products may then be used in conventional DPIs for pulmonary or nasal delivery, dispersed in propellants for use in pMDIs, or the particles may be reconstituted in water for use in nebulizers.

In some instances, it may be desirable to add an inert carrier to the spray-dried material to improve the metering properties of the final product. This may especially be the case when the spray dried powder is very small (less than about 5 microns) or when the intended dose is extremely small, whereby dose metering becomes difficult. In general, such carrier particles (also known as bulking agents) are too large to be delivered to the lung and simply impact the mouth and throat and are swallowed. Such carriers typically consist of sugars such as lactose, mannitol, or trehalose. Other inert materials, including polysaccharides and cellulosics, may also be useful as carriers.

Spray-dried powders comprising nanoparticulate benzodiazepine, such as lorazepam, may used in conventional DPIs, dispersed in propellants for use in pMDIs, or reconstituted in a liquid media for use with nebulizers.

### F. Freeze-Dried Nanoparticulate Compositions

For a benzodiazepine that is denatured or destabilized by heat, such as having a low melting point (i.e., about 70 to about 150 degrees C.), or, for example, biologics, sublimation is preferred over evaporation to obtain a dry powder nanoparticulate composition. This is because sublimation avoids the high process temperatures associated with spray-drying. In addition, sublimation, also known as freeze-drying or lyophilization, can increase the shelf stability of a benzodiazepine, particularly for biological products. Freeze-dried particles can also be reconstituted and used in nebulizers. Aggregates of freeze-dried nanoparticulate benzodiazepine, such as lorazepam, can be blended with either dry powder intermediates or used alone in DPIs and pMDIs for either nasal or pulmonary delivery.

Sublimation involves freezing the product and subjecting the sample to strong vacuum conditions. This allows for the formed ice to be transformed directly from a solid state to a vapor state. Such a process is highly efficient and, therefore, provides greater yields than spray-drying. The resultant freeze-dried product contains benzodiazepine, such as lorazepam, and at least one surface stabilizer. The benzodiazepine, such as lorazepam, is typically present in an aggregated state and can be used for inhalation alone (either pulmonary or nasal), in conjunction with diluent materials (lactose, mannitol, etc.), in DPIs or pMDIs, or reconstituted for use in a nebulizer.

### IV. Method of Treatment

In human therapy, it is important to provide a benzodiazepine, such as lorazepam, dosage form that delivers the required therapeutic amount of the drug *in vivo,* and that renders the drug bioavailable in a constant manner. Thus, another aspect of the present invention provides a method of treating a mammal, including a human, requiring status epilepticus treatment, irritable bowel syndrome treatment, sleep induction, acute psychosis, or pre-anesthesia medication using a nanoparticulate benzodiazepine, such as lorazepam, formulation of the invention. Such methods comprise the step of administering to a subject a therapeutically effective amount of a nanoparticulate benzodiazepine, such as lorazepam, formulation of the present invention. In one embodiment, the nanoparticulate benzodiazepine, such as lorazepam, formulation is an injectable formulation. In another embodiment, the nanoparticulate benzodiazepine, such as lorazepam, formulation is an aerosol formulation. Particularly advantageous features of the present invention include that the pharmaceutical formulation of the invention does not require the presence of polyethylene glycol and propylene glycol as stabilizers. In addition, the injectable formulation of the invention can provide a high lorazepam concentration in a small volume to be injected. A general protocol for injectable administration comprises a bolus injection of a benzodiazepine, such as lorazepam, with one continuous fast injection, rather than a slow infusion of the drug.

The benzodiazepine, such as lorazepam, compositions of the invention can be used for pulmonary or intranasal delivery. Pulmonary and intranasal delivery are particularly useful for the delivery of benzodiazepine, and preferably, lorazepam which is difficult to deliver by other routes of administration. Pulmonary or intranasal delivery is effective both for systemic delivery and for localized delivery to treat diseases of the air cavities.

The aerosols of the present invention, both aqueous and dry powder, are particularly useful in the treatment of respiratory-related illnesses such as asthma, emphysema, respiratory distress syndrome, chronic bronchitis, cystic fibrosis, chronic obstructive pulmonary disease, organ-transplant rejection, tuberculosis and other infections of the lung, fugal infections, respiratory illness associated with acquired immune deficiency syndrome, oncology, and systemic administration of an anti-emetic, analgesic, cardiovascular agent, *etc.* The formulations and method result in improved lung and nasal surface area coverage by the administered benzodiazepine, such as lorazepam.

In addition, the aerosols of the invention, both aqueous and dry powder, can be used in a method for diagnostic imaging. Such a method comprises administering to the body of a test subject in need of a diagnostic image an effective contrast-producing amount of the nanoparticulate aerosol diagnostic image contrast composition. Thereafter, at least a portion of the body containing the administered contrast agent is exposed to x-rays or a magnetic field to produce an x-ray or magnetic resonance image pattern corresponding to the presence of the contrast agent. The image pattern can then be visualized.

"Therapeutically effective amount" is used herein with respect to a drug dosage, shall mean that dosage that provides the specific pharmacological response for which the drug is administered in a significant number of subjects in need of such treatment. It is emphasized that 'therapeutically effective amount,' administered to a particular subject in a particular instance will not always be effective in treating the diseases described herein, even though such dosage is deemed a "therapeutically effective amount" by those skilled in the art. "Therapeutically effective amount" also includes an amount that is effective for prophylaxis. It is to be further understood that drug dosages are, in particular instances, measured as oral dosages, or with reference to drug levels as measured in blood.

One of ordinary skill will appreciate that effective amounts of a benzodiazepine, such as lorazepam, can be determined empirically and can be employed in pure form or, where such forms exist, in pharmaceutically acceptable salt, ester, or prodrug form. Actual dosage levels of benzodiazepine, such as lorazepam, in the aerosol and injectable compositions of the invention may be varied to obtain an amount of benzodiazepine, such as lorazepam, that is effective to obtain a desired therapeutic response for a particular composition and method of administration. The selected dosage level therefore depends upon the desired therapeutic effect, the route of administration, the potency of the administered benzodiazepine, such as lorazepam" the desired duration of treatment, and other factors.

Dosage unit compositions may contain such amounts of such submultiples thereof as may be used to make up the daily dose. It will be understood, however, that the specific dose level for any particular patient will depend upon a variety of factors: the type and degree of the cellular or physiological response to be achieved; activity of the specific agent or composition employed; the specific agents or composition employed; the age, body weight, general health, sex, and diet of the patient; the time of administration, route of administration, and rate of excretion of the agent; the duration of the treatment; drugs used in combination or coincidental with the specific agent; and like factors well known in the medical arts.

It will be apparent to those skilled in the art that various modifications and variations can be made in the compositions, methods, and uses of the present invention without departing from the spirit or scope of the invention. Thus, it is intended that the present invention cover the modifications and variations of this invention provided they come within the scope of the appended claims and their equivalents.

The following prophetic example is given to illustrate the present invention. It should be understood, however, that the spirit and scope of the invention is not to be limited to the specific conditions or details described in this example but should only be limited by the scope of the claims that follow. All references identified herein, including U.S. patents, are hereby expressly incorporated by reference.

### Example 1

The purpose of this example was to prepare a nanoparticulate benzodiazepine, such as lorazepam, formulation.

An aqueous dispersion of 10% (w/w) lorazepam, combined with 2% (w/w) polyvinylpyrrolidone (PVP) K29/32 and 0.05% (w/w) dioctylsulfosuccinate (DOSS), could be milled in a 10 ml chamber of a NanoMill® 0.01 (NanoMill Systems, King of Prussia, PA; see e.g., U.S. Patent No. 6,431,478), along with 500 micron PolyMill® attrition media (Dow Chemical Co.) (89% media load). In an exemplary process, the mixture could be milled at a speed of 2500 rpms for 60 minutes.

Following milling, the particle size of the milled lorazepam particles can be measured, in deionized distilled water, using a Horiba LA 910 particle size analyzer. The initial mean milled lorazepam particle size is expected to be less than 2000 nm.

The following pages 57 to 64 contain preferred embodiments. Accordingly, the term "claim" as used therein refers to such a "preferred embodiment".
1. A nanoparticulate composition comprising:
   (a) a benzodiazepine having an effective average particle size of less than about 2000 nm, wherein the benzodiazepine is selected from the group consisting of alprazolam, brotizolam, chlordiazepoxide, clobazam, clonazepam, clorazepam, demoxazepam, flumazenil, flurazepam, halazepam, midazolam, nordazepam, medazepam, diazepam, nitrazepam, oxazepam, midazepam, lorazepam, prazepam, quazepam, triazolam, temazepam, loprazolam, pharmaceutically acceptable salts and esters thereof, and mixtures thereof; and
   (b) at least one surface stabilizer.
2. The composition of claim 1, wherein the surface stabilizer is selected from the group consisting of a nonionic surfactant, an ionic surfactant, a cationic surfactant, an anionic surfactant, and a zwitterionic surfactant.
3. The composition of claim 1 or claim 2, wherein the surface stabilizer is selected from the group consisting of hypromellose, hydroxypropylcellulose, polyvinylpyrrolidone, sodium lauryl sulfate, dioctylsulfosuccinate, gelatin, casein, lecithin, dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, polyoxyethylene stearates, colloidal silicon dioxide, phosphates, carboxymethylcellulose calcium, carboxymethylcellulose sodium, methylcellulose, hydroxyethylcellulose, hypromellose phthalate, noncrystalline cellulose, magnesium aluminum silicate, triethanolamine, polyvinyl alcohol, tyloxapol, poloxamers, poloxamines, Tetronic 1508®, an alkyl aryl polyether sulfonate, a mixture of sucrose stearate and sucrose distearate, p-isononylphenoxypoly-(glycidol), Crodestas SL-40® (Croda, Inc.); and SA9OHCO, decanoyl-N-methylglucamide; n-decyl (-D-glucopyranoside; n-decyl (-D-maltopyranoside; n-dodecyl (-D-glucopyranoside; n-dodecyl (-D-maltoside; heptanoyl-N-methylglucamide; n-heptyl-(-D-glucopyranoside; n-heptyl (-D-thioglucoside; n-hexyl (-D-glucopyranoside; nonanoyl-N-methylglucamide; n-noyl (-D-glucopyranoside; octanoyl-N-methylglucamide; n-octyl-(-D-glucopyranoside; octyl (-D-thioglucopyranoside; PEG-phospholipid, PEG-cholesterol, PEG-cholesterol derivative, PEG-vitamin A, PEG-vitamin E, lysozyme, random copolymers of vinyl pyrrolidone and vinyl acetate, cationic polymers, cationic biopolymers, cationic polysaccharides, cationic cellulosics, cationic alginates, cationic phospholipids, cationic nonpolymeric compounds, poly-n-methylpyridinium, anthryul pyridinium chloride, cationic phospholipids, chitosan, polylysine, polyvinylimidazole, polybrene, polymethylmethacrylate trimethylammoniumbromide bromide, hexyldesyltrimethylammonium bromide, polyvinylpyrrolidone-2-dimethylaminoethyl methacrylate dimethyl sulfate, cationic lipids, sulfonium, phosphonium, quarternary ammonium compounds, stearyltrimethylammonium chloride, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium bromide, C12-15dimethyl hydroxyethyl ammonium chloride, C12-15dimethyl hydroxyethyl ammonium bromide, coconut dimethyl hydroxyethyl ammonium chloride, coconut dimethyl hydroxyethyl ammonium bromide, myristyl trimethyl ammonium methyl sulfate, lauryl dimethyl benzyl ammonium chloride, lauryl dimethyl benzyl ammonium bromide, lauryl dimethyl (ethenoxy)4 ammonium chloride, lauryl dimethyl (ethenoxy)4 ammonium bromide, N-alkyl (C12-18)dimethylbenzyl ammonium chloride, N-alkyl (C14-18)dimethyl-benzyl ammonium chloride, N-tetradecylidmethylbenzyl ammonium chloride monohydrate, dimethyl didecyl ammonium chloride, N-alkyl and (C12-14) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C12-14) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C12,C15,C17 trimethyl ammonium bromides, dodecylbenzyl triethyl ammonium chloride, poly-diallyldimethylammonium chloride, dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MIRAPOL, ALKAQUAT, alkyl pyridinium salts, amines, alkylamines, dialkylamines, alkanolamines, polyethylenepolyamines, N,N-dialkylaminoalkyl acrylates, vinyl pyridine, amine salts, lauryl amine acetate, stearyl amine acetate, alkylpyridinium salt, alkylimidazolium salt, amine oxides, imide azolinium salts, protonated quaternary acrylamides, methylated quaternary polymers, and cationic guar.
4. The composition of any one of claims 1 to 3, wherein the nanoparticulate benzodiazepine particles have an effective average particle size selected from the group consisting of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 650 nm, less than about 600 nm, less than about 550 nm, less than about 500 nm, less than about 450 nm, less than about 400 nm, less than about 350 nm, less than about 300 nm, less than about 250 nm, less than about 200 nm, less than about 150 nm, less than about 100 nm, less than about 75 nm, and less than about 50 nm.
5. The composition of any one of claims 1 to 4, wherein the composition is formulated into an aerosol of an aqueous dispersion of the composition of claim 1, wherein essentially each droplet of the aerosol comprises at least one nanoparticulate benzodiazepine particle, wherein:
   (a) the benzodiazepine has a solubility in the aqueous dispersion of less than about 10 mg/mL; and
   (b) the droplets of the aerosol have a mass median aerodynamic diameter (MMAD) less than or equal to about 100 microns.
6. The aerosol composition of claim 5, wherein the benzodiazepine is present in a concentration selected from the group consisting of from about 0.05 mg/mL up to about 600 mg/mL, about 10 mg/mL or more, about 100 mg/mL or more, about 200 mg/mL or more, about 400 mg/mL or more, and about 600 mg/mL.
7. The aerosol composition of claim 5 or claim 6, wherein the composition is suitable for administration of the benzodiazepine dosage in about 15 seconds or less.
8. The aerosol composition of any one of claims 5 to 7, wherein the droplets of the aerosol have a mass median aerodynamic diameter (MMAD) selected from the group consisting of about 2 to about 10 microns, about 2 to about 6 microns, less than about 2 microns, about 5 to about 100 microns, and about 30 to about 60 microns.
9. The composition of any one of claims 1 to 4, formulated into an injectable composition.
10. The injectable composition of claim 9, comprising as a surface stabilizer a povidone polymer.
11. The injectable composition of claim 10, wherein the povidone polymer has a molecular weight of about 40,000 daltons or less.
12. The injectable composition of any one of claims 9 to 11, wherein the effective average particle size of the benzodiazepine particles is less than about 600 nm.
13. A method of treating a subject in need comprising administering to the subject a nanoparticulate benzodiazepine composition comprising:
   (a) a benzodiazepine having an effective average particle size of less than about 2000 nm, wherein the benzodiazepine is selected from the group consisting of alprazolam, brotizolam, chlordiazepoxide, clobazam, clonazepam, clorazepam, demoxazepam, flumazenil, flurazepam, halazepam, midazolam, nordazepam, medazepam, diazepam, nitrazepam, oxazepam, midazepam, lorazepam, prazepam, quazepam, triazolam, temazepam, loprazolam, pharmaceutically acceptable salts and esters thereof, and mixtures thereof; and
   (b) at least one surface stabilizer.
14. The method of claim 13, wherein the surface stabilizer is selected from the group consisting of a nonionic surfactant, an ionic surfactant, a cationic surfactant, an anionic surfactant, and a zwitterionic surfactant.
15. The method of claim 13 or claim 14, wherein the surface stabilizer is selected from the group consisting of hypromellose, hydroxypropylcellulose, polyvinylpyrrolidone, sodium lauryl sulfate, dioctylsulfosuccinate, gelatin, casein, lecithin, dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, polyoxyethylene stearates, colloidal silicon dioxide, phosphates, carboxymethylcellulose calcium, carboxymethylcellulose sodium, methylcellulose, hydroxyethylcellulose, hypromellose phthalate, noncrystalline cellulose, magnesium aluminum silicate, triethanolamine, polyvinyl alcohol, tyloxapol, poloxamers, poloxamines, Tetronic 1508®, an alkyl aryl polyether sulfonate, a mixture of sucrose stearate and sucrose distearate, p-isononylphenoxypoly-(glycidol), Crodestas SL-40® (Croda, Inc.); and SA9OHCO, decanoyl-N-methylglucamide; n-decyl (-D-glucopyranoside; n-decyl (-D-maltopyranoside; n-dodecyl (-D-glucopyranoside; n-dodecyl (-D-maltoside; heptanoyl-N-methylglucamide; n-heptyl-(-D-glucopyranoside; n-heptyl (-D-thioglucoside; n-hexyl (-D-glucopyranoside; nonanoyl-N-methylglucamide; n-noyl (-D-glucopyranoside; octanoyl-N-methylglucamide; n-octyl-(-D-glucopyranoside; octyl (-D-thioglucopyranoside; PEG-phospholipid, PEG-cholesterol, PEG-cholesterol derivative, PEG-vitamin A, PEG-vitamin E, Iysozyme, random copolymers of vinyl pyrrolidone and vinyl acetate, cationic polymers, cationic biopolymers, cationic polysaccharides, cationic cellulosics, cationic alginates, cationic phospholipids, cationic nonpolymeric compounds, poly-n-methylpyridinium, anthryul pyridinium chloride, cationic phospholipids, chitosan, polylysine, polyvinylimidazole, polybrene, polymethylmethacrylate trimethylammoniumbromide bromide, hexyldesyltrimethylammonium bromide, polyvinylpyrrolidone-2-dimethylaminoethyl methacrylate dimethyl sulfate, cationic lipids, sulfonium, phosphonium, quarternary ammonium compounds, stearyltrimethylammonium chloride, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium bromide, C12-15dimethyl hydroayethyl ammonium chloride, C12-15dimethyl hydroxyethyl ammonium bromide, coconut dimethyl hydroxyethyl ammonium chloride, coconut dimethyl hydroxyethyl ammonium bromide, myristyl trimethyl ammonium methyl sulfate, lauryl dimethyl benzyl ammonium chloride, lauryl dimethyl benzyl ammonium bromide, lauryl dimethyl (ethenoxy)4 ammonium chloride, lauryl dimethyl (ethenoxy)4 ammonium bromide, N-alkyl (C12-18)dimethylbenzyl ammonium chloride, N-alkyl (C14-18)dimethyl-benzyl ammonium chloride, N-tetradecylidmethylbenzyl ammonium chloride monohydrate, dimethyl didecyl ammonium chloride, N-alkyl and (C12-14) dimethyl 1-napthylmethyl ammonium chloride, trimethylammonium halide, alkyl-trimethylammonium salts, dialkyl-dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N-tetradecyldimethylbenzyl ammonium, chloride monohydrate, N-alkyl(C12-14) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C12, C15, C17 trimethyl ammonium bromides, dodecylbenzyl triethyl ammonium chloride, poly-diallyldimethylammonium chloride, dimethyl ammonium chlorides, alkyldimethylammonium halogenides, tricetyl methyl ammonium chloride, decyltrimethylammonium bromide, dodecyltriethylammonium bromide, tetradecyltrimethylammonium bromide, methyl trioctylammonium chloride, POLYQUAT, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MIRAPOL, ALKAQUAT, alkyl pyridinium salts, amines, alkylamines, dialkylamines, alkanolamines, polyethylenepolyamines, NN-dialkylaminoalkyl acrylates, vinyl pyridine, amine salts, lauryl amine acetate, stearyl amine acetate, alkylpyridinium salt, alkylimidazolium salt, amine oxides, imide azolinium salts, protonated quaternary acrylamides, methylated quaternary polymers, and cationic guar.
16. The method of any one of claims 13 to 15, wherein the nanoparticulate benzodiazepine particles have an effective average particle size selected from the group consisting of less than about 1900 nm, less than about 1800 nm, less than about 1700 nm, less than about 1600 nm, less than about 1500 nm, less than about 1400 nm, less than about 1300 nm, less than about 1200 nm, less than about 1100 nm, less than about 1000 nm, less than about 900 nm, less than about 800 nm, less than about 700 nm, less than about 650 nm, less than about 600 nm, less than about 550 nm, less than about 500 nm, less than about 450 nm, less than about 400 nm, less than about 350 nm, less than about 300 nm, less than about 250 nm, less than about 200 nm, less than about 150 nm, less than about 100 nm, less than about 75 nm, and less than about 50 nm.
17. The method of any one of claims 13 to 16, wherein the composition is formulated into an aerosol of an aqueous dispersion of the composition of claim 1, wherein essentially each droplet of the aerosol comprises at least one nanoparticulate benzodiazepine particle, wherein:
   (a) the benzodiazepine has a solubility in the aqueous dispersion of less than about 10 mg/mL; and
   (b) the droplets of the aerosol have a mass median aerodynamic diameter (MMAD) less than or equal to about 100 microns.
18. The method of claim 17, wherein the benzodiazepine is present in a concentration selected from the group consisting of from about 0.05 mg/mL up to about 600 mg/mL, about 10 mg/mL or more, about 100 mg/mL or more, about 200 mg/mL or more, about 400 mg/mL or more, and about 600 mg/mL.
19. The method of claim 17 or claim 18, wherein the composition is suitable for administration of the benzodiazepine dosage in about 15 seconds or less.
20. The method of any one of claims 17 to 19, wherein the droplets of the aerosol have a mass median aerodynamic diameter (MMAD) selected from the group consisting of about 2 to about 10 microns, about 2 to about 6 microns, less than about 2 microns, about 5 to about 100 microns, and about 30 to about 60 microns.
21. The method of any one of claims 13 to 16, wherein the composition is formulated into an injectable dosage form.
22. The method of claim 21, comprising as a surface stabilizer a povidone polymer.
23. The method of claim 22, wherein the povidone polymer has a molecular weight of about 40,000 daltons or less.
24. The method of any one of claims 21 to 23, wherein the effective average particle size of the benzodiazepine particles is less than about 600 nm.

## Claims

1. A composition comprising:
(a) particles of a benzodiazepine having an effective average particle size of less than about 2000 nm, wherein the benzodiazepine is selected from the group consisting of clobazam, pharmaceutically acceptable salts and esters thereof, and mixtures thereof; and
(b) at least one surface stabilizer adsorbed to, or associated with, the surface of the benzodiazepine particles.

2. A composition according claim 2 wherein the surface stabilizer is selected from the group consisting of hypromellose, hydroxypropylcellulose, polyvinylpyrrolidone, sodium lauryl sulfate, dioctylsulfosuccinate, gelatin, casein, lecithin, dextran, gum acacia, cholesterol, tragacanth, stearic acid, benzalkonium chloride, calcium stearate, glycerol monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, polyoxyethylene stearates, colloidal silicon dioxide, phosphates, carboxymethylcellulose calcium, carboxymethylcellulose sodium, methylcellulose, hydroxyethylcellulose, hypromellose phthalate, noncrystalline cellulose, magnesium aluminum silicate, triethanolamine, polyvinyl alcohol, tyloxapol, poloxamers, poloxamines, Tetronic 1508®, an alkyl aryl polyether sulfonate, a mixture of sucrose stearate and sucrose distearate, p-isononylphenoxypoly-(glycidol), Crodestas SL40 ® (Croda, Inc.); and SA90HCO, decanoyl-N-methylglucamide; n-decyl (-D-glucopyranoside; n-decyl (-D-maltopyranoside; n-dodecyl (-D-glucopyranoside; n-dodecyl (-D-maltoside; heptanoyl-N-methylglucamide; n-heptyl(-D-glucopyranoside; n-heptyl(-D-thioglucoside; n-hexyl (-D-glucopyranoside; nonanoyl-N-methylglucamide; n-noyl (-D-glucopyranoside; octanoyl-N-methylglucamide; n-octyl-(-D-glucopyranoside; octyl ( D-thioglucopyranoside; PEG-phospholipid, PEG-cholesterol, PEG-cholesterol derivative, PEG- vitamin A, PEG-vitamin E, lysozyme, random copolymers of vinyl pyrrolidone and vinyl acetate, cationic polymers, cationic biopolymers, cationic polysaccharides, cationic cellulosics, cationic alginates, cationic phospholipids, cationic nonpolymeric compounds, poly-n-methylpyridinium, anthryul pyridinium chloride, cationic phospholipids, chitosan, polylysine, polyvinylimidazole, polybrene, polymethylmethacrylate trimethylammoniumbromide bromide, hexyldesyltrimethylammonium bromide, polyvinylpyrrolidone-2- dimethylaminoethyl methacrylate dimethyl sulfate, cationic lipids, sulfonium, phosphonium, quarternary ammonium compounds, stearyltrimethylammonium chloride, benzyl-di(2-chloroethyl)ethylammonium bromide, coconut trimethyl ammonium chloride, coconut trimethyl ammonium bromide, coconut methyl dihydroxyethyl ammonium chloride, coconut methyl dihydroxyethyl ammonium bromide, decyl triethyl ammonium chloride, decyl dimethyl hydroxyethyl ammonium chloride, decyl dimethyl hydroxyethyl ammonium bromide, C12-15dimethyl hydroxyethyl ammonium chloride, C12-15dimethyl hydroxyethyl ammonium bromide, coconut dimethyl hydroxyethyl ammonium chloride, coconut dimethyl hydroxyethyl ammonium bromide, myristyl trimethyl ammonium methyl sulfate, lauryl dimethyl benzyl ammonium chloride, lauryl dimethyl benzyl ammonium bromide, lauryl dimethyl (ethenoxy)4 ammonium chloride, lauryl dimethyl (ethenoxy)4 ammonium bromide, N-alkyl (C12-18)dimethylbenzyl ammonium chloride, N-alkyl (C14-18)dimethyl benzyl ammonium chloride, N-tetradecylidmethylbenzyl ammonium chloride monohydrate, dimethyl didecyl ammonium chloride, N-alkyl and (C 12-14) dimethyl 1-napthylmethyl ammonium chloride, trimethylammonium halide, alkyl-trimethylammonium salts, dialkyl- dimethylammonium salts, lauryl trimethyl ammonium chloride, ethoxylated alkyamidoalkyldialkylammonium salt, an ethoxylated trialkyl ammonium salt, dialkylbenzene dialkylammonium chloride, N-didecyldimethyl ammonium chloride, N- tetradecyldimethylbenzyl ammonium, chloride monohydrate, N-alkyl(C12-14) dimethyl 1-naphthylmethyl ammonium chloride, dodecyldimethylbenzyl ammonium chloride, dialkyl benzenealkyl ammonium chloride, lauryl trimethyl ammonium chloride, alkylbenzyl methyl ammonium chloride, alkyl benzyl dimethyl ammonium bromide, C12, C15, C17 trimethyl ammonium bromides, dodecylbenzyl triethyl ammonium chloride, poly-diallyldimethylammonium chloride, dimethyl ammonium chlorides, alkyldimethylammoniurn halogenides, tricetyl methyl ammonium chloride, decyltrimethylammonium bromide, dodecyltriethylammonium bromide, tetradecyltrimethylammonium bromide, methyl trioctylammonium chloride, POLYQUAT, tetrabutylammonium bromide, benzyl trimethylammonium bromide, choline esters, benzalkonium chloride, stearalkonium chloride compounds, cetyl pyridinium bromide, cetyl pyridinium chloride, halide salts of quaternized polyoxyethylalkylamines, MIRAPOL, ALKAQUAT, alkyl pyridinium salts, amines, alkylamines, dialkylamines, alkanolamines, polyethylenepolyamines, N,N-dialkylaminoalkyl acrylates, vinyl pyridine, amine salts, lauryl amine acetate, stearyl amine acetate, alkylpyridinium salt, alkylimidazolium salt, amine oxides, imide azolinium salts, protonated quaternary acrylamides, methylated quaternary polymers, and cationic guar.

3. A composition according to either of claims 1 and 2 wherein the surface stabilizer is selected from the group consisting of povidone (polyvinylpyrrolidone), hydroxypropyl methylcellulose (hypromellose), dioctylsulfosuccinate, sodium lauryl sulfate, hydroxypropyl cellulose, block copolymers of ethylene oxide and propylene oxide (poloxamers), polyoxyethylene sorbitan fatty acid esters, tyloxapol, polyethoxylated castor oil derivatives, polyoxyethylene alykyl ethers (macrogol ethers), gelatin, lecithin (phosphatides), lysozyme,

4. A composition according to any one of claims 1 to 3 which is sterile or comprises a preservative.

5. A composition according to claim 4 comprising a preservative selected from the group consisting of potassium sorbate, methylparaben, propylparaben, benzoic acid and its salts, butylparaben, ethyl alcohol, butyl alcohol, phenol and benzalkonium chloride.

6. A composition according to any one of claims 1 to 4 formulated into an aerosol or into an injectable composition.

7. A composition according to any one of claims 1 to 4 wherein the composition is formulated as a dispersion of benzodiazepine particles in an aqueous vehicle which upon being aerosolized produces an aerosol in which essentially each droplet of the aerosol comprises at least one benzodiazepine particle, wherein:
(a) the benzodiazepine has a solubility in the aqueous dispersion of less than about 10 mg/mL; and
(b) the droplets of the aerosol have a mass median aerodynamic diameter (MMAD) less than or equal to about 100 microns.

8. A composition according to either of claims 6 and 7 wherein the benzodiazepine is present in a concentration selected from the group consisting of from about 0.05 mg/mL up to about 600 mg/mL, about 10 mg/mL or more, about 100 mg/mL or more, about 200 mg/mL or more, about 400 mg/mL or more, and about 600 mg/mL.

9. A composition according to any one of claims 6 to 8 wherein the composition is suitable for administration of the benzodiazepine dosage in about 15 seconds or less.

10. A composition according to any one of claims 7 to 9 wherein the droplets of the aerosol have a mass median aerodynamic diameter (MMAD) selected from the group consisting of about 2 to about 10 microns, about 2 to about 6 microns, less than about 2 microns, about 5 to about 100 microns, and about 30 to about 60 microns.

11. A composition according to any one of claims 1 to 4 formulated into an injectable composition wherein the effective average particle size of the benzodiazepine particles is less than about 600 nm.

12. A composition according to claim 11 wherein the surface stabilizer is a povidone (polyvinylpyrrolidone) polymer having a molecular weight of 40,000 daltons or less.

13. A composition according to claim 1 for use in the treatment of status epilepticus, irritable bowel syndrome, sleep induction and acute psychosis wherein the treatment comprises administering a therapeutically effective amount of an injectibable or aerosol nanoparticulate benzodiazepine composition to a subject in need of such treatment.
